# EUROPEAN PATENT APPLICATION

(11) **EP 1 283 267 A1**
(43) Date of publication of application: **12.02.2003**
(21) Application number: 01930150.6
(22) Date of filing: 15.05.2001
(51) Int. Cl.: C12N 15/63, C12N 1/21, C12Q 1/02, C12M 1/00, A61K 45/00, A61P 31/12

(54) **VECTOR FOR ANALYSING REPLICATION MECHANISM OF RNA VIRUS AND USE THEREOF**

(30) Priority: 15.05.2000 JP 2000142451
(71) Applicant: Tokyo Metropolitan Organization for Medical Research, Shinjuku-ku, Tokyo 163-8001 (JP); CHUGAI SEIYAKU KABUSHIKI KAISHA, Tokyo, 115-8543 (JP)
(72) Inventor: KOHARA, Michinori, Kita-ku, Tokyo 114-0014 (JP); MATSUZAKI, Junichi, Gotenba-shi, Shizuoka 412-8513 (JP); OKAMOTO, Kouichi, Gotenba-shi, Shizuoka 412-8513 (JP); KATSUME,Asao, c/o Chugai Seiyaku Kabushiki Kaisha, Gotenba-shi, Shizuoka 412-8513 (JP)
(74) Representative: Maschio, Antonio
(86) International application number: JP0104033
(87) International publication number: WO01088161

(57) **Abstract**

The object of the present invention is to reproduce conditions where viral proteins can function along with the original life cycle of the viruses and to establish an expression system which can evaluate the function of viral proteins by a reporter gene without generating infectious viruses. The present invention provides an assay system comprising a vector containing DNA coding for RNA virus-derived RNA-dependent RNA polymerase and a vector containing a reporter gene expressed in the anti-sense direction.

SEQUENCE LISTING

<110> CHUGAI SEIYAKU KABUSHIKI KAISHA
TOKYO METROPOLITAN ORGANIZATION FOR MEDICAL RESEARCH

<120> A VECTOR FOR ANALYSIS OF REPRODUCTION SYSTEM OF RNA VIRUS AND ITS
USE

<130> PH-1187-PCT

<150> JP 2000-142451
<151> 2000-05-15

<160> 17

<170> PatentIn Ver. 2.0

<210> 1
<211> 328
<212> DNA
<213> Hepatitis C Virus

<400> 1
ccgggtacca cccttgcgag tctggagaca tcgggccaga agtgtccgcg ctaagctgct 60
gtcccagggg gggagggctg ccacttgtgg taagtacctc ttcaactggg cagtaaggac 120
caagctcaaa ctcactccaa tcccggcagc gtcccagttg gacttgtcca gctggttcgt 180
ggctggttac agcgggggag acatatatca cagcctgtct cgtgcccgac cccgctggtt 240
catgttgtgc ctactcctac tttcagtagg ggtaggcatc tacctgctcc ccaaccgata 300
ataatctaga tttaaataag cttaagtt 328

<210> 2

<211> 304

<212> DNA

<213> Hepatitis C Virus

<400> 2

ggaattcatt taaatctcga gtaatacgac tcactatagg gcgtagaccg tgcatcatga 60

gcacaaatcc taaaccccaa agaaaaacca aacgtaacac caaccgccgc ccacaggacg 120

tcaagttccc gggtggtggt cagatcgttg gtggagttta cctgttgccg cgcaggggcc 180

ccaggttggg tgtgcgcgcg actaggaaga cttccgagcg gtcacaacct cgtggaaggc 240

gacaacctat ccccaaggct cgccagcccg agggcagggc ctgggctcag cccgggtacc 300

accc 304

<210> 3

<211> 186

<212> DNA

<213> Hepatitis C Virus

<400> 3

aaactcgagt aatacgactc actatagggc caccatggct cccatcacgg cctattccca 60

acagacgcgg ggcctacttg gttgcatcat cactagcctc acaggccggg acaaaaacca 120

agtcgagggg gaggttcaag tggtctccac cgcgacacaa tccttcctgg cgacctgcgt 180

caatgg 186

<210> 4

<211> 412

<212> DNA

<213> Hepatitis C Virus

<400> 4

aactgcagaa gctttaatac gactcactat agccagcccc cgattggggg cgacactcca 60

ccatagatca ctcccctgtg aggaactact gtcttcacgc agaaagcgtc tagccatggc 120

gttagtatga gtgtcgtgca gcctccagga ccccccctcc cgggagagcc atagtggtct 180

gcggaaccgg tgagtacacc ggaattgcca ggacgaccgg gtcctttctt ggatcaaccc 240

gctcaatgcc tggagatttg ggcgtgcccc cgcgagactg ctagccgagt agtgttgggt 300

cgcgaaaggc cttgtggtac tgcctgatag ggtgcttgcg agtgccccgg gaggtctcgt 360

agaccgtgca tcatggaaga cgccaaaaac ataaagaaag gcccggcgcc at 412

<210> 5

<211> 393

<212> DNA

<213> Hepatitis C Virus

<400> 5

gctctagaca gggccagccc ccgattgggg gcgacactcc accatagatc actcccctgt 60

gaggaactac tgtcttcacg cagaaagcgt ctagccatgg cgttagtatg agtgtcgtgc 120

agcctccagg accccccctc ccgggagagc catagtggtc tgcggaaccg gtgagtacac 180

cggaattgcc aggacgaccg ggtcctttct tggatcaacc cgctcaatgc ctggagattt 240

gggcgtgccc ccgcgagact gctagccgag tagtgttggg tcgcgaaagg ccttgtggta 300

ctgcctgata gggtgcttgc gagtgccccg ggaggtctcg tagaccgtgc atcatggaag 360

acgccaaaaa cataaagaaa ggcccggcgc cat 393

<210> 6

<211> 863

<212> DNA

<213> Hepatitis C Virus

<400> 6

gtagaccgtg catcatgagc acaaatccta aaccccaaag aaaaaccaaa cgtaacacca 60

acattgaaca agatggattg cacgcaggtt ctccggccgc ttgggtggag aggctattcg 120

gctatgactg ggcacaacag acaatcggct gctctgatgc cgccgtgttc cggctgtcag 180

cgcaggggcg cccggttctt tttgtcaaga ccgacctgtc cggtgccctg aatgaactgc 240

aggacgaggc agcgcggcta tcgtggctgg ccacgacggg cgttccttgc gcagctgtgc 300

tcgacgttgt cactgaagcg ggaagggact ggctgctatt gggcgaagtg ccggggcagg 360

atctcctgtc atctcacctt gctcctgccg agaaagtatc catcatggct gatgcaatgc 420

ggcggctgca tacgcttgat ccggctacct gcccattcga ccaccaagcg aaacatcgca 480

tcgagcgagc acgtactcgg atggaagccg gtcttgtcga tcaggatgat ctggacgaag 540

agcatcaggg gctcgcgcca gccgaactgt tcgccaggct caaggcgcgc atgcccgacg 600

gcgaggatct cgtcgtgacc catggcgatg cctgcttgcc gaatatcatg gtggaaaatg 660

gccgcttttc tggattcatc gactgtggcc ggctgggtgt ggcggaccgc tatcaggaca 720

tagcgttggc tacccgtgat attgctgaag agcttggcgg cgaatgggct gaccgcttcc 780

tcgtgcttta cggtatcgcc gctcccgatt cgcagcgcat cgccttctat cgccttcttg 840

acgagttctt ctgataatct aga 863

<210> 7

<211> 17

<212> DNA

<213> Artificial Sequence

<220>

<223> forward primer for PCR

<400> 7

ccgggtacca cccttgc 17

<210> 8

<211> 46

<212> DNA

<213> Artificial Sequence

<220>

<223> reverse primer for PCR

<400> 8

aacttaagct tatttaaatc tagattatta tcggttgggg agcagg 46

<210> 9

<211> 60

<212> DNA

<213> Artificial Sequence

<220>

<223> forward primer for PCR

<400> 9

ggaattcatt taaatctcga gtaatacgac tcactatagg gcgtagaccg tgcatcatga 60

<210> 10

<211> 16

<212> DNA

<213> Artificial Sequence

<220>

<223> reverse primer for PCR

<400> 10

gggtggtacc cgggct                                                    16

<210> 11

<211> 57

<212> DNA

<213> Artificial Sequence

<220>

<223> forward primer for PCR

<400> 11

aaactcgagt aatacgactc actatagggc caccatggct cccatcacgg cctattc     57

<210> 12

<211> 20

<212> DNA

<213> Artificial Sequence

<220>

<223> reverse primer for PCR

<400> 12

ccattgacgc aggtcgccag                                                20

<210> 13

<211> 49

<212> DNA

<213> Artificial Sequence

<220>

<223> forward primer for PCR

<400> 13

aactgcagaa gctttaatac gactcactat agccagcccc cgattgggg 49

<210> 14

<211> 57

<212> DNA

<213> Artificial Sequence

<220>

<223> reverse primer for PCR

<400> 14

atggcgccgg gcctttcttt atgtttttgg cgtcttccat gatgcacggt ctacgag 57

<210> 15

<211> 27

<212> DNA

<213> Artificial Sequence

<220>

<223> forward primer for PCR

<400> 15

gctctagaca gggccagccc ccgattg 27

<210> 16

<211> 85

<212> DNA

<213> Artificial Sequence

<220>

<223> forward primer for PCR

<400> 16

gtagaccgtg catcatgagc acaaatccta aaccccaaag aaaaaccaaa cgtaacacca 60

acattgaaca agatggattg cacgc 85

<210> 17

<211> 33

<212> DNA

<213> Artificial Sequence

<220>

<223> reverse primer for PCR

<400> 17

tctagattat cagaagaact cgtcaagaag gcg 33

## Description

### Technical Field

The present invention relates to a vector for analyzing the replication mechanism of RNA viruses. This vector enables the evaluation of activity of RNA virus-derived RNA-dependent RNA polymerase (hereinafter this may be simply referred to as "RNA polymerase") based on the expression level of a reporter gene by taking the interaction between viral proteins and host factors based on the original life cycle of viruses into consideration. Thus, this vector is useful for elucidating the replication mechanism of RNA viruses and functions of viral gene products, developing therapeutic agents and therapeutic techniques, and the like.

### Background Art

Hepatitis C virus (hereinafter referred to as "HCV") is a major causative virus of non-A, non-B hepatitis after blood transfusion, and cDNA of its gene was cloned in 1989. Various studies have been heretofore made on HCV using cloned cDNA, and socially important achievements such as, in particular, prevention of infection and a diagnostic method have been established. Thus, HCV infection after blood transfusion is rarely observed these days. However, the number of HCV-infected patients worldwide is estimated to be as much as several percent of the entire world population. This virus-derived infection is likely to cause a prolonged chronic disease, and is known to be very highly likely to induce chronic hepatitis, followed by hepatic cirrhosis, and hepatic carcinoma. Thus, complete elimination of HCV after infection is a very important issue.

Interferon (IFN) therapy is widely employed for treating chronic hepatitis C, however its availability is only about 30%. Because of problems including frequent side effects such as fever and the high cost of drugs successful results have not been attained. The availability is expected to be enhanced since the types, dosage forms, and doses of IFN have been studied, and consensus IFN was developed. Treatment through the combined use of IFN and an antiviral agent such as ribavirin has been attempted, however, none of those has yet become a reliable treating method.

On the other hand, a search for an anti-HCV agent based on the life cycle of HCV has been attempted in many research institutes using HCV cDNA. In particular, searches for a pharmaceutical have been conducted based on selective inhibition of a function of viral proteins by targeting protease (NS3) coded in a non-structure region, RNA-dependent RNA polymerase (NS5B), and the like, preparing recombinant proteins thereof, and evaluating the inhibition of enzyme activity. At present, however, no therapeutic agent for HCV has been developed from an inhibitor found through such research.

As an assay system for pharmaceutical screening, the *in vitro* cell infection system is useful. However, although there are several reports on such kinds of infection systems, a system which is reproducible, stable, and practical has not yet been developed because the amount replicated is low, etc. The cell infection system involves generation of infectious viruses and, thus, it lacks convenience in terms of biohazard and the like.

From our studies, it was considered that a viral protein generated by the original replication of viruses does not exhibit its function by itself in the cell, and instead, a plurality of viral proteins function as a complex including host factors, and the structure of the functional complex is not necessarily congruous with that of each of the isolated proteins. In the case of HCV, an inhibitor in an assay system using purified enzymes has not successfully led to the development of a therapeutic agent because of the structural disjunction between the state of the complex and the state of purified enzymes. Thus, it was considered important to construct a cell replication system which more faithfully imitates the state of actual viral replication. In that case, a cell assay system which does not generate infectious viruses and which can detect the function of viral proteins by a reporter gene would be useful for elucidating the replication mechanism of viruses and functions of viral gene products, developing therapeutic agents and therapeutic techniques, and the like.

An object of the present invention is to establish an expression system which can reproduce conditions where viral proteins can function along with the original life cycle of the viruses and evaluate the function of viral proteins by a reporter gene without generating infectious viruses.

### Disclosure of the Invention

We have conducted concentrated studies in order to attain the above object. As a result, we found that introduction of a vector expressing RNA polymerase and a vector expressing a reporter gene in the anti-sense direction into a host cell enables the evaluation of RNA polymerase activity in a host based on the amount of the expression product of the reporter gene. This led to the completion of the present invention.

More specifically, the present invention includes the inventions according to 1 to 19 below.
1. A vector for analyzing the replication mechanism of RNA viruses, which comprises DNA coding for RNA virus-derived RNA-dependent RNA polymerase.
2. A vector for analyzing the replication mechanism of RNA viruses, which comprises a reporter gene expressed in the anti-sense direction.
3. A vector for analyzing the replication mechanism of RNA viruses, which comprises DNA coding for RNA virus-derived RNA-dependent RNA polymerase and a reporter gene expressed in the anti-sense direction.
4. A kit for analyzing the replication mechanism of RNA viruses, which comprises the vector for analyzing the replication mechanism of RNA viruses according to 1 and the vector for analyzing the replication mechanism of RNA viruses according to 2.
5. A method for evaluating RNA polymerase activity, wherein a vector comprising DNA coding for RNA virus-derived RNA-dependent RNA polymerase and a vector comprising DNA corresponding to an entry site of the RNA polymerase and a reporter gene located upstream of the DNA and expressed in the anti-sense direction are produced, these vectors are introduced into a host cell, and the amount of the expression product of the reporter gene in the cell is measured, thereby evaluating RNA polymerase activity.
6. A method for evaluating RNA polymerase activity, wherein a vector comprising DNA coding for RNA virus-derived RNA-dependent RNA polymerase, DNA corresponding to an entry site of the RNA polymerase, and a reporter gene located upstream of the DNA corresponding to the entry site and expressed in the anti-sense direction is produced, this vector is introduced into a host cell, and the amount of the expression product of the reporter gene in the cell is measured, thereby evaluating RNA polymerase activity.
7. A method for screening a therapeutic agent for viral diseases, wherein a vector comprising DNA coding for RNA virus-derived RNA-dependent RNA polymerase and a vector comprising DNA corresponding to an entry site of the RNA polymerase and a reporter gene located upstream of the DNA and expressed in the anti-sense direction are produced, these vectors are introduced into a host cell, test substances are inoculated into this cell, and the amount of the expression product of the reporter gene in the cell is measured, and a substance which can increase or decrease the amount of the expression product of the reporter gene is selected from the test substances.
8. A method for screening a therapeutic agent for viral diseases, wherein a vector comprising DNA coding for RNA virus-derived RNA-dependent RNA polymerase, DNA corresponding to an entry site of the RNA polymerase, and a reporter gene located upstream of the DNA corresponding to the entry site and expressed in the anti-sense direction is produced, this vector is introduced into a host cell, test substances are inoculated into this cell, and the amount of the expression product of the reporter gene in the cell is measured, and a substance which can increase or decrease the amount of the expression product of the reporter gene is selected from the test substances.
9. A substance, which is obtained by the method for screening a therapeutic agent for viral diseases according to 7 or 8.
10. A cell, which comprises the vector according to 1 and the vector according to 2.
11. A cell, which comprises the vector according to 3.
12. A method for evaluating the replication capacity of RNA viruses, which comprises the steps of:
   (1) expressing RNA virus-derived RNA-dependent RNA polymerase in a host;
   (2) bringing RNA having a reporter gene located so as not to be expressed in the absence of RNA polymerase into contact with the RNA virus-derived RNA-dependent RNA polymerase; and
   (3) measuring the expression level of the expression product of the reporter gene.
13. A method for evaluating the replication capacity of RNA viruses, which comprises the steps of:
   (1) expressing RNA virus-derived RNA-dependent RNA polymerase in a host;
   (2) expressing RNA coding for a reporter gene in the anti-sense direction;
   (3) synthesizing RNA coding for the reporter gene in the sense direction by the RNA polymerase; and
   (4) measuring the amount of the expression product of the reporter gene.
14. A method for evaluating the replication capacity of RNA viruses, which comprises the steps of:
   (1) expressing a reporter gene incorporated in a first vector in the anti-sense direction and RNA coding for RNA-dependent RNA polymerase gene incorporated in a second vector identical to or different from the first vector in the sense direction in a host;
   (2) expressing RNA-dependent RNA polymerase from the RNA;
   (3) synthesizing RNA coding for the reporter gene in the sense direction by the RNA-dependent RNA polymerase; and
   (4) measuring the amount of the expression product of the reporter gene.
15. A method for screening an inhibitor against viral replication comprising addition of a step of bringing a test substance into contact to the evaluation method according to 12 to 14.
16. The vector according to 1 to 3, wherein the RNA virus is HCV.
17. The evaluation method according to 5, 6, and 12 to 14, wherein the RNA virus is HCV.
18. The screening method according to 7, 8, and 15, wherein the RNA virus is HCV.
19. The cell according to 10 or 11, wherein the RNA virus is HCV.

This specification includes part or all of the contents as disclosed in the specification and/or drawings of Japanese Patent Application No. 2000-142451, which is a priority document of the present application.

### Brief Description of the Drawings

Fig. 1 is a diagram showing a summary of the evaluation of RNA polymerase activity by a trans-acting vector.
Fig. 2 is a diagram showing a summary of the evaluation of RNA polymerase activity by a cis-acting vector.
Fig. 3 is a diagram showing a process for constructing a pE2 vector (first half).
Fig. 4 is a diagram showing a process for constructing a pE2 vector (last half).
Fig. 5 is a diagram showing a process for constructing a pE1b vector.
Fig. 6 is a diagram showing a process for constructing a pE1 vector.
Fig. 7 is a diagram showing a process for constructing a pIFG0 vector.
Fig. 8 is a diagram showing a process for constructing a pRL1 vector.
Fig. 9 is a diagram showing a process for constructing a pRL1b vector.
Fig. 10 is a diagram showing a process for constructing a pRL2b vector.
Fig. 11 is a diagram showing a process for constructing a pRL3b vector.
Fig. 12 is a diagram showing a process for constructing a pRL4b vector (first half).
Fig. 13 is a diagram showing a process for constructing a pRL4b vector (last half).
Fig. 14 is a diagram showing a process for constructing a pEL2 vector.

### Embodiment for Carrying out the Invention

The present invention will hereinafter be described in detail.

### (1) Vector for analyzing replication mechanism of RNA viruses

The vector for analyzing the replication mechanism of RNA viruses according to the present invention includes the three types of vectors of the following (a) to (c).
(a) A vector which is introduced into a host cell together with the vector according to (b) below, and comprises DNA coding for virus-derived RNA-dependent RNA polymerase.
(b) A vector which is introduced into a host cell together with the vector according to (a) above, and comprises a reporter gene expressed in the anti-sense direction (hereinafter this may be simply referred to as "anti-sense reporter gene").
(c) A vector which comprises DNA coding for a virus-derived RNA-dependent RNA polymerase and a reporter gene expressed in the anti-sense direction.

In general, a sequence in the direction from an initiation codon to a termination codon of the gene coding for a protein is a sense sequence, and a sequence in the reverse direction is an anti-sense sequence. The term "anti-sense direction" used herein means that, based on the structure of the expression vector, transcription is carried out in the anti-sense direction upon expression by introduction into the cell, that is, a reporter gene is inserted in such a direction that a reverse mRNA is synthesized first. Accordingly, mRNA produced from the expression vector cannot produce a functional reporter protein. Instead, a functional reporter protein is produced upon synthesis of complementary RNA by the RNA-dependent RNA polymerase using the mRNA as a template.

A vector according to (a) may be any vector as long as it can express DNA coding for the RNA virus-derived RNA-dependent RNA polymerase in a host cell, and a plasmid is preferably used. RNA polymerase to be used is not particularly limited and RNA polymerase derived from various viruses can be used. More specifically, HCV-derived RNA polymerase (NS5B), Poliovirus-derived RNA polymerase, Rotavirus-derived RNA polymerase, and the like can be used. Among these, use of HCV-derived RNA polymerase is preferred. A promoter to be used is not particularly limited. For example, CAG promoter, CMV promoter, T7 promoter, EF1α promoter, SV40 early promoter, and SV40 late promoter can be used. Especially, use of T7 promoter, CAG promoter, and the like, which allow a large amount of RNA to be synthesized, is preferred. Regarding DNA coding for virus-derived RNA-dependent RNA polymerase, only a DNA region corresponding to a protein having enzyme activity may be incorporated into a vector. Alternatively, a region connected to this DNA region may also be incorporated into the vector. For example, in the case of HCV, the NS5B protein has RNA polymerase activity, however, DNA coding for this protein as well as DNA coding for NS5A, NS4, NS3, NS2, E2, E1, or Core protein, which is adjacent to NS5B protein, may also be incorporated into the vector. Incorporation of regions other than the region coding for RNA polymerase into the vector enables the evaluation of RNA polymerase activity in the environment where the interaction between RNA polymerase and other factors can be considered. A vector may initiate the expression simultaneously with introduction, or, it may initiate the expression upon specific operation such as the Cre-loxP system (Nat Sternberg et al., J. Molecular Biology 150. p467-486 1981, Nat Sternberg et al., J. Molecular Biology 150. p487-507 1981).

A vector according to (b) may be any vector as long as a transcript of the anti-sense reporter gene can be synthesized in a host cell, and a plasmid is preferably used. A promoter to be used is not particularly limited. For example, CAG promoter, CMV promoter, T7 promoter, EF1α promoter, SV40 early promoter, and SV40 late promoter can be used. Especially, use of T7 promoter, CAG promoter, and the like, which allow a large amount of RNA to be synthesized, is preferred. A reporter gene may be any gene as long as the amount of the expression product can be measured by one means or another. Examples of such a gene include the luciferase gene, the chloramphenicol acetyl transferase gene, and the β-galactosidase gene, and use of the luciferage gene is especially preferred.

Even if both the vector according to (a) and the vector according to (b) have been introduced into a host cell, the expression product of the reporter gene is not synthesized unless the RNA polymerase expressed from the vector according to (a) binds to a suitable position in the transcript of the vector according to (b). Accordingly, DNA which corresponds to an entry site of RNA polymerase expressed from the vector according to (a) should be generally inserted downstream of the anti-sense reporter gene of the vector according to (b). As described later, when these vectors are used for identifying the entry site of RNA polymerase, DNA corresponding to the entry site is not necessarily inserted. The term "entry site of RNA polymerase" used herein refers to a specific site in RNA to which the RNA virus-derived RNA-dependent RNA polymerase binds and a site from which RNA synthesis is initiated.

In general, the RNA-dependent RNA polymerase is an enzyme that synthesizes complementary RNA using RNA as a template. Thus, the origin should be recognized when initiating RNA synthesis. In a conventional technique, a suitable primer is set as an artificial origin of replication and RNA is synthesized therefrom. In the present invention, the RNA virus-derived RNA-dependent RNA polymerase binds to a site (sequence) which is originally recognized by the RNA polymerase and RNA synthesis is initiated, and exhibits activity. This sequence is referred to as "an entry site of RNA polymerase" herein.

In order to realize internal ribosome entry site (IRES)-dependent translation, DNA corresponding to IRES is preferably inserted downstream of the reporter gene. IRES may be derived from the same type of virus as the RNA polymerase to be used, however, use of IRES with higher translation activity is preferable. Examples of such IRES include: vascular endothelial growth factor (VEGF) IRES (SP163) (Miller, D.L. et al., FEBS Lett 1998 Sep 4; 434(3): 417-20); Encephalomyocarditis virus (EMCV) IRES (Jang, S.K. et al., Journal of Virology, 62(8): 2636-43, 1988 Aug.); and Poliovirus IRES (Ishii, T. et al., J. Gen. Virol. (1999), 80(4), 917-920; Ehrenfeld, E. and Semler, B.L., Curr. Top. Microbiol. Immunol. (1995), 203, 65-83; Klinck, R. et al., Nucleic. Acids Res. (1997), 25(11), 2129-2137; Johansen, L.K. and Morrow, C.D., Virology (2000), 273(2), 391-399). The reporter gene in this vector is inserted so as to be expressed in the anti-sense direction and, thus, the transcript of the reporter gene in the sense direction is not be generated originally. In fact, however, a non-specific transcript is synthesized in the upstream direction. The transcript in the sense direction disadvantageously increases the background value of the present evaluation method. Accordingly, a reversed poly (A) signal is preferably inserted downstream of DNA corresponding to the entry site in order to prevent the transcript in the sense direction from being synthesized.

A vector according to (c) may be any vector as long as it can express DNA coding for RNA polymerase and can synthesize the transcript of the anti-sense reporter gene in a host cell, and a plasmid is preferably used. This vector can be produced in the same manner as the vector according to (a) and the vector according to (b) except that DNA coding for RNA polymerase and the anti-sense reporter gene are included in the same vector.

As described later, the vector for analyzing the replication mechanism of RNA viruses according to the present invention is mainly used to evaluate RNA polymerase activity derived from RNA viruses. This enables the analysis of the replication mechanism of RNA viruses. In addition, it can be utilized in applications such as the following.

### 1) Measurement of translation activity of IRES

The expression product of the reporter gene is translated in an IRES-dependent manner. Accordingly, various IRES (or a sequence expected to be IRES) are incorporated into a vector according to (b) or (c) to measure the amount of the expression product of the reporter gene. Thus, the protein expression inductive activity of each IRES can be measured.

### 2) Identification of viral protein associated with RNA polymerase

The RNA virus-derived RNA polymerase does not necessarily exhibit its activity by itself. Instead, it sometimes exhibits its activity upon formation of an enzyme complex through binding to other factors. Alternatively, it may be produced as a precursor and processed with protease and the like, thereby exhibiting activity for the first time. For example, the HCV-derived NS5B protein is considered to form a complex with an adjacent NS protein, and is processed with NS3, i.e., protease.

As is apparent from the foregoing, DNA coding for various viral proteins other than RNA polymerase is incorporated into the vector according to (a) or (c) to examine whether or not the expression product of the reporter gene is generated. Thus, a factor associated with RNA polymerase can be identified.

### 3) Identification of entry site of RNA polymerase

The RNA virus-derived RNA polymerase binds to a specific site in RNA (an entry site) and initiates RNA synthesis. Accordingly, the entry site of the RNA polymerase can be identified by inserting anti-sense reporter genes into various sites in the vector according to (b) or (c) and investigating whether or not the expression product of the reporter gene is generated. For example, the entry site of the HCV-derived NS5B is 3'UTR of HCV genomic RNA. Thus, the expression product of the reporter gene is generated only when the anti-sense reporter gene is inserted upstream of DNA corresponding to 3'UTR.

### 4) Identification of host factor associated with RNA polymerase

The RNA virus-derived RNA polymerase is considered to form a complex not only with a factor of the virus itself but also with a host factor. Accordingly, the vectors according to (a) and (b) or the vector according to (c) are introduced into various host cells, and whether or not the expression product of the reporter gene is generated in each cell is investigated. This enables the identification of a host factor associated with the RNA polymerase.

The present invention provides a kit for analyzing the replication mechanism of RNA viruses comprising the vectors according to (a) and (b). Use of the kit according to the present invention enables the evaluation of RNA polymerase activity by introducing both vectors according to (a) and (b) into the host cell and measuring the amount of the expression product of the reporter gene in the cell. Further, test substances may be inoculated in the cell into which both vectors according to (a) and (b) have been introduced, and a substance which increases or decreases the amount of the expression product of the reporter gene in the cell may be selected. Thus, a test substance which could be a therapeutic agent for viral diseases can be screened.

### (2) Method for evaluating RNA polymerase activity

According to the method for evaluating RNA polymerase activity of the present invention, RNA polymerase activity is evaluated by preparing vectors according to the (a) and (b)' below or a vector according to (c)' below, introducing these vectors into host cells, and measuring the amount of the expression product of the reporter gene in the cell. In the present invention, the RNA virus for which replication mechanism can be analyzed by evaluating RNA polymerase activity, may be any RNA virus as long as it has RNA as genome. Examples thereof include, but are not limited to, HCV, poliovirus, and rotavirus, which present problems as causative viruses of viral diseases, and HCV can be specifically exemplified. The vectors according to (a) and (b)' are hereinafter referred to as "trans-acting vectors" and the vector according to (c)' is referred to as a "cis-acting vector."
(a) A vector that comprises DNA coding for RNA virus-derived RNA-dependent RNA polymerase.
(b)' A vector that comprises DNA corresponding to the entry site of the RNA polymerase and a reporter gene located upstream of said DNA and expressed in the anti-sense direction.
(c)' A vector that comprises DNA coding for virus-derived RNA-dependent RNA polymerase, DNA corresponding to the entry site of the RNA polymerase, and a reporter gene located upstream of the DNA corresponding to the entry site and expressed in the anti-sense direction.

The vectors according to (b)' and (c)' can be produced in the similar manner as the vectors according to (b) and (c) above.

Host cells usable herein include, but are not limited to, primary hepatocytes such as IMY, HuH-7, HepG2, MOLT-4, MT-2, and Daudi, other hepatocytes, and a hemocyte-derived cell. As a method for transducing a vector into a host, a commonly used technique in the art, such as the calcium phosphate method, can be used without any particular limitation.

The amount of the expression product in the host cell may be measured depending on the type of reporter gene used.

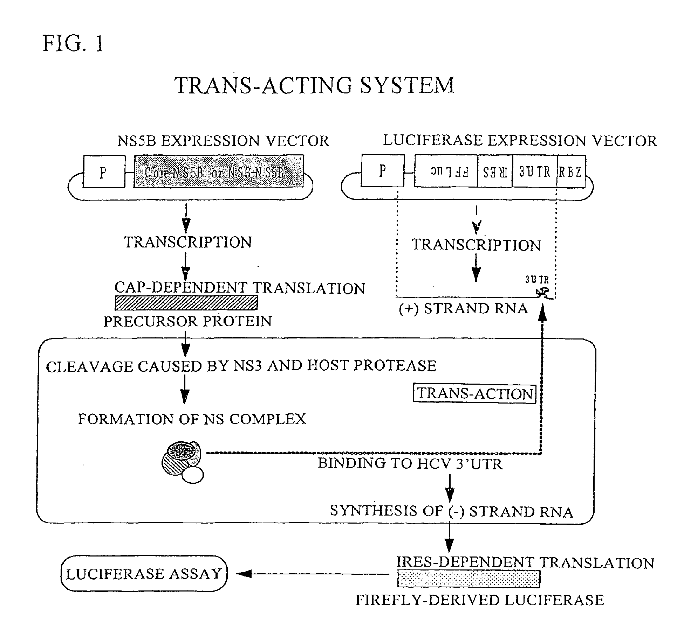

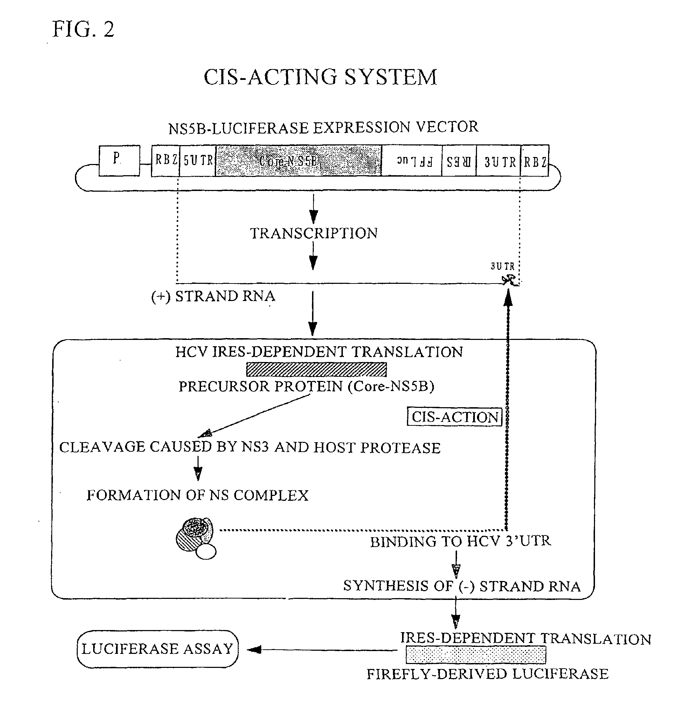

Hereinafter, the method for evaluating RNA polymerase activity when the NS5B protein of HCV is used as RNA polymerase and the luciferase gene is used as the reporter gene is described with reference to Fig. 1 and Fig. 2.

Fig. 1 shows a summary of the evaluation of RNA polymerase activity by the trans-acting vector.

The NS5B expression vector (at left in the diagram) comprises DNA coding for HCV protein, Core-NS5B or NS3-NSSB. A precursor protein is synthesized from the vector through cap-dependent translation. The precursor protein is cleaved into a plurality of proteins (NS proteins) by the NS3 protein, which is protease, and a host protease. The cleaved protein(s) and host factor(s) form an enzyme complex containing NS5B.

On the other hand, a luciferase expression vector (at right in the diagram) comprises the luciferase gene in the anti-sense direction, IRES (the anti-sense direction), DNA corresponding to 3'UTR of HCV genomic RNA, and the like. RNA containing the luciferase transcript in the anti-sense direction, IRES, and 3'UTR is synthesized from this vector.

The entry site of NS5B is 3'UTR of HCV genomic RNA. Thus, the enzyme complex comprising NSSB binds to 3'UTR of RNA derived from the luciferase expression vector and uses this RNA as a template to synthesize complementary RNA. This complementary RNA is translated in an IRES-dependent manner, and luciferase is then synthesized. The amount of this complementary RNA corresponds to enzyme activity of the enzyme complex containing NS5B and the amount of this RNA corresponds to the amount of the synthesized luciferase. Thus, enzyme activity of the enzyme complex containing NS5B corresponds to the amount of luciferase.

Fig. 2 shows a summary of the evaluation of RNA polymerase activity by the cis-acting vector.

The NS5B-luciferase expression vector comprises DNA corresponding to 5'UTR, DNA coding for the Core-NS5B protein, the luciferase gene in the anti-sense direction, IRES (the anti-sense direction), DNA corresponding to 3'UTR, and the like, and RNA corresponding to these DNA is synthesized through transcription. This RNA is translated in an IRES-dependent manner and an enzyme complex containing NS5B is formed, as with the case of the trans-acting vector. This enzyme complex binds to 3'UTR of the RNA mentioned above (i.e., RNA that is an origin of the enzyme complex itself) and synthesizes RNA which is complementary to this RNA. This complementary RNA is translated in an IRES-dependent manner and luciferase is synthesized. The amount of the synthesized luciferase corresponds to the activity of RNA polymerase of the enzyme complex, as with the case of the trans-acting vector.

The method for evaluating RNA polymerase activity can be utilized in screening of a therapeutic agent for viral diseases. A candidate substance as a therapeutic agent for viral diseases is inoculated in the cell into which the above vector has been introduced, and the amount of the expression product of the reporter gene in the cell is measured. This enables judgment of whether or not the inoculated substance inhibits the virus-derived RNA polymerase activity. Specifically, a substance which inhibits virus-derived RNA polymerase activity in accordance with the method of the present invention can be an effective and novel therapeutic agent for viral diseases.

Accordingly, the present invention can provide a substance obtained by the method for screening a therapeutic agent for viral diseases.

The present invention also provides a method for evaluating the replication capacity of RNA viruses, comprising the steps of:
(1) expressing RNA virus-derived RNA-dependent RNA polymerase in a host;
(2) bringing RNA having a reporter gene located so as not to be expressed in the absence of the RNA polymerase into contact with the RNA virus-derived RNA-dependent RNA polymerase; and
(3) measuring the expression level (amount) of the expression product of the reporter gene.

The above-described method enables the evaluation of RNA virus-derived RNA-dependent RNA polymerase activity based on the expression level of the expression product of a reporter gene. This enables evaluation of the replication capacity of RNA viruses. For example, by comparing an RNA virus with an unknown RNA polymerase activity to an RNA virus with a known RNA polymerase activity, or comparing a mutant virus to a wild-type virus, the replication capacity can be found to be relatively higher or lower.

In the method for locating the reporter gene so as not to be expressed in the absence of the RNA polymerase, for example, a vector comprising a reporter gene for expressing in the anti-sense direction may be used. In this case, RNA which codes for the reporter gene in the anti-sense direction is expressed from the vector utilizing a general transcription mechanism in the host. Accordingly, when the RNA-dependent RNA polymerase is absent, a peptide which is encoded by the reporter gene is not expressed. Only when the RNA polymerase is present, RNA which codes for a reporter gene in the sense direction is synthesized and a peptide encoded by the reporter gene expressed. The genes coding for the RNA polymerase may be incorporated in the sense direction into the same vector as the vector comprising a reporter gene to be expressed in the anti-sense direction. Alternatively, it may be incorporated in the sense direction into a vector different from the vector comprising the reporter gene.

The present invention can further provide a method for screening a virus replication inhibitor using the method for evaluating the replication capacity of viruses. This method can be accomplished by adding a step of bringing a test substance into contact to the method for evaluating the replication capacity of viruses.

### Examples

The present invention is described below in more detail with reference to the examples, although the technical scope of the present invention is not limited to these examples.

### [Example 1]

### (1) Construction of enzyme expression vector

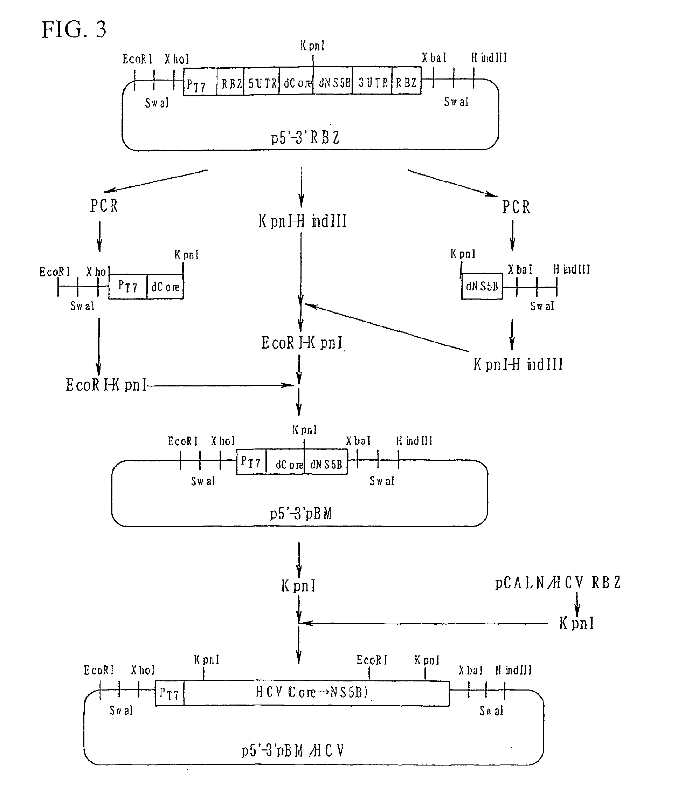

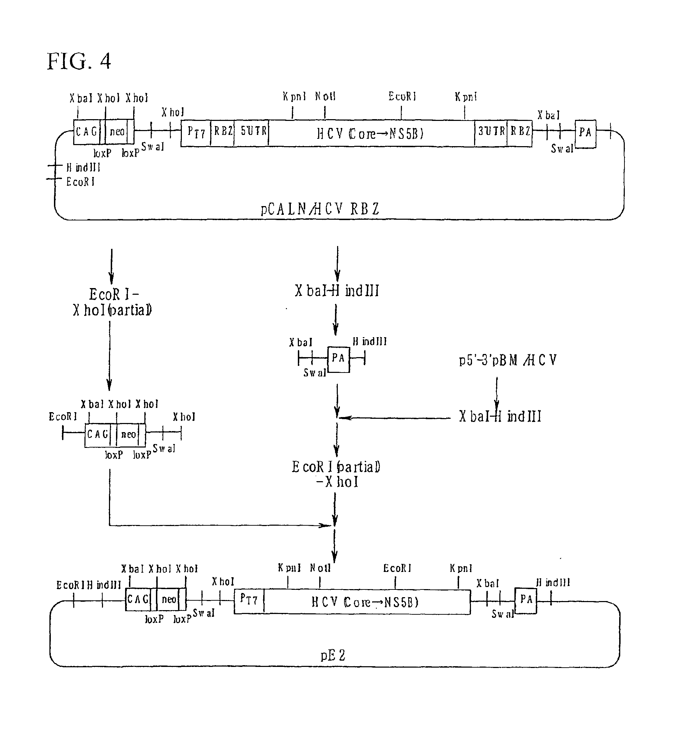

### 1) Construction of pE2 (Fig. 3 and Fig. 4) and pE2b

An expression vector pE2 for the full length of a HCV translated region in which the 5' terminal un-translated region of the HCV genome (5'UTR), the 3' terminal un-translated region of the HCV genome (3'UTR), and ribozyme sequences at both terminuses are removed from pCALN/HCV RBZ (a microorganism containing this vector has been deposited at the International Patent Organism Depositary of the National Institute of Advanced Industrial Science and Technology (Tsukuba Central 6, 1-1-1 Higashi, Tsukuba, Ibaraki, Japan) as of June 21, 1999 under the accession number of FERM BP-6763) was constructed in the manner described below.

### Construction of p5'-3' pBM

At the outset, PCR was carried out using p5'-3'RBZ (this vector is prepared by removing the Cre-loxP expression site and the poly(A) site from the pCALN/HCV RBZ) as a template in order to remove ribozyme sequences at 3'UTR and on the 3' terminus side. Nucleotide sequences of PCR primers used are as follows.

HCV Fw1: 5'-CCG GGT ACC ACC CTT GC-3' (SEQ ID NO: 7)

HCV Rv1: 5'-AAC TTA AGC TTA TTT AAA TCT AGA TTA TTA TCG GTT
GGG GAG CAG G-3' (SEQ ID NO: 8)

The PCR reaction solution was prepared by adding 10 µL of 10x Pyrobest Buffer, 8 µL of 25 mM dNTP mixture, 1 µL each of 100 µM primers, 1 µL of 55 ng/µL template (p5'-3'RBZ), and 0.5 µL of 5 units/µL Pyrobest DNA Polymerase (Takara Shuzo Co., Ltd.) to a tube and adjusted to 100 µL with the aid of sterilized water. In PCR, heating at 94°C for 5 minutes was first carried out, and a cycle of denaturation at 98°C for 10 seconds, annealing at 60°C for 30 seconds, and elongation at 72°C for 1 minute was then repeated 30 times. The amplified sequence is shown in SEQ ID NO: 1. Subsequently, this PCR product was subjected to 1.5% agarose electrophoresis, and a specifically amplified fragment of interest was extracted from a gel. The fragment of interest was extracted in the following manner using QIAquick Gel Extraction Kit (QIAGEN). A gel was first cleaved out, a 3x amount of QG Buffer was added, and incubation was carried out at 50°C for 10 minutes to dissolve the gel. After the incubation, an amount of isopropanol equivalent to the amount of gel was added and mixed. The mixture was transferred to QIAquick spin column and centrifuged. The flow-through fraction was removed, and 750 µL of PE Buffer was added to the QIAquick spin column for washing, followed by centrifugation. The flow-through fraction was removed, and centrifugation was again carried out to completely remove the PE Buffer. The QIAquick spin column was transferred into a 1.5 mL tube, sterilized water was added, and the mixture was centrifuged to elute DNA (hereinafter a DNA fragment was extracted from a gel thoroughly in accordance with this method). Subsequently, this DNA fragment was double-digested with *Hind*III and *Kpn*I. p5'-3'RBZ was also double-digested with *Hind*III and *Kpn*I. The reacted solution was then subjected to agarose electrophoresis. For the PCR product, a 0.3 kbp DNA fragment was extracted from a gel, and for p5'-3'RBZ, a 3.7 kbp DNA fragment was extracted from a gel. Extraction was carried out in the same manner as described above using QIAquick Gel Extraction Kit (QIAGEN). Subsequently, these two DNA fragments were ligated in the following manner using Rapid DNA Ligation Kit (Boeheringer Mannheim). 3.7 µL of p5'-3'RBZ DNA fragment, 0.3 µL of DNA fragment of the PCR product, 1 µL of 5x DNA dilution buffer, 5 µL of 2x DNA ligation buffer, and 0.5 µL of 5 units/µL T4 DNA Ligase were mixed, and the mixture was subjected to ligation at room temperature for 30 minutes (hereinafter, all ligation was carried out using this kit in this manner). After completion of the reaction, transformation into a competent cell of *Escherichia coli* DH5α strain was carried out using the reaction solution. More specifically, transformation was carried out as follows. The solution after reaction was added to the competent cell in an amount of 1/10 (v/v), allowed to stand on ice for 30 minutes, subjected to heat shock at 42°C for 45 seconds, allowed to stand on ice again for 2 minutes, and a 5x amount of SOC medium was then added thereto. The total amount of the mixture was inoculated on an LB+Amp plate (1% Bactotrypton, 0.5% yeast extract, 1% sodium chloride, 1.5% agar, 100 µg/mL ampicillin) and cultured overnight at 37°C (hereinafter, all transformation was carried out in this manner). The next day, the colonies developed on the plate were shake-cultured overnight in a tube containing 3 ml of LB+Amp medium (1% Bactotrypton, 0.5% yeast extract, 1% sodium chloride, 75 µg/mL ampicillin) at 37°C. Thereafter, the culture solution was centrifuged to harvest, and plasmid DNA was prepared by mini-preparation using QIAprep Spin Plasmids Kits (QIAGEN). Mini-preparation was carried out in the following manner. At first, 250 µL of P1 Buffer was added and suspended in a cell pellet, and 250 µL of P2 Buffer was then added and mixed. Subsequently, 350 µL of N3 Buffer was added and mixed, followed by centrifugation, and the supernatant was transferred into QIAprep spin column and then centrifuged. The flow-through fraction was removed, 750 µL of PE Buffer was added to the QIAprep spin column for washing, centrifugation was carried out, the flow-through fraction was removed, and centrifugation was carried out again to completely remove the PE Buffer. The contents of the QIAprep spin column was transferred to a 1.5 mL tube, 50 µL of sterilized water was added, and the mixture was centrifuged to elute plasmid DNA (hereinafter, all mini-preparation of plasmid DNA was carried out in this manner). Regarding this plasmid DNA, the nucleotide sequence of the insertion fragment corresponding to SEQ ID NO: 1 was subjected to a sequence reaction using the Dye Terminator Cycle Sequencing FS Ready Reaction Kit (Perkin Elmer) in accordance with the protocol attached to the kit, followed by sequence analysis. The procedure is shown below. As the solution for the sequence reaction, 8 µL of Terminator Ready Reaction Mix, 6 µL of 0.1 µg/µL Template DNA, and 3.2 µL of 1 pmol/µL Primer were mixed and adjusted to 20 µL with the aid of sterilized water. The reaction was carried out at 96°C for 5 minutes, at 96°C for 30 seconds, at 50°C for 15 seconds, and at 60°C for 4 minutes, and this cycle was repeated 25 times. The reacted solution was then transferred into a 1.5 ml tube, 50 µL of 100% ethanol and 2 µL of 3M sodium acetate (pH 4.6) were added thereto and mixed, and the mixture was allowed to stand on ice for 10 minutes. The mixture was centrifuged and the supernatant was removed. Thereafter, 250 µL of 70% ethanol was added and the mixture was centrifuged again. The supernatant was removed, and after air-drying, 20 µL of migration buffer was added to dissolve a sample. The mixture was then subjected to nucleotide sequence analysis by auto-sequencer Model 310 (Perkin Elmer) (hereinafter, all sequence reactions and nucleotide sequence analyses were carried out in this manner). As a result, the analyzed sequence was found to be an expected sequence. Thus, this fragment was designated as p3'pBM and used below.

Subsequently, PCR was carried out using p5'-3'RBZ as a template to construct a vector in which 5'UTR and ribozyme sequences at on the 5' terminus side have been removed from p3'pBM. The nucleotide sequences of PCR primers used are as follows.

HCV Fw2: 5'-GGA ATT CAT TTA AAT CTC GAG TAA TAC GAC TCA CTA
TAG GGC GTA GAC CGT GCA TCA TGA-3' (SEQ ID NO: 9)

HCV Rv2: 5'-GGG TGG TAC CCG GGC T-3' (SEQ ID NO: 10)

In PCR, 25 cycles of the same reaction as above were carried out using Pyrobest DNA Polymerase (Takara Shuzo Co., Ltd.). The amplified sequence is shown in SEQ ID NO: 2. The PCR product was subjected to electrophoresis, and a specifically amplified fragment of interest was extracted from a gel. Subsequently, this DNA fragment was double-digested with *Kpn*I and *Eco*RI. p3'pBM was also double-digested with *Kpn*I and *Eco*RI. The reacted solution was subjected to agarose electrophoresis. For the PCR product, a 0.3 kbp DNA fragment was extracted from a gel, and for p5'RBZ, a 3.4 kbp DNA fragment was extracted from a gel. Subsequently, these two DNA fragments were ligated. *Escherichia coli* DH5α strain was transformed with the ligated fragment, inoculated on an LB+Amp plate, and cultured at 37°C overnight. The colonies developed on the plate were shake-cultured in a tube containing 3 mL of LB+Amp medium at 37°C overnight. The culture solution was centrifuged to harvest, and plasmid DNA was prepared by mini-preparation. Whether or not a DNA fragment was inserted into a vector was investigated through a restriction enzyme treatment, and plasmid DNA for which insertion of a DNA fragment had been confirmed was analyzed for the nucleotide sequence of a portion corresponding to SEQ ID NO: 2, that is, the insertion fragment. As a result, the analyzed sequence was confirmed to be the expected sequence. Thus, this fragment was designated as p5'-3'pBM and used in the following experiment.

### Construction of p5'-3'pBM/HCV

The vector p5'-3'pBM/HCV in which an 8.5 kbp DNA fragment from the *Kpn*I site on the core gene in the HCV translated region to the *Kpn*I site on the NSSB gene was incorporated into the *Kpn*I site of p5'-3'pBM was constructed in the following manner. At the outset, p5'-3'pBM and pCALN/HCV RBZ were digested with the restriction enzyme reaction solution *Kpn*I and subjected to agarose electrophoresis, and a 3.7 kbp linearized plasmid of p5'-3'pBM and an 8.5 kbp DNA fragment of pCALN/HCV RBZ were extracted from a gel. Subsequently, p5'-3'pBM digested with *Kpn*I was subjected to alkaline phosphatase treatment using the BAPping Kit (TOYOBO). Treatment was in accordance with the protocol attached to the kit (hereinafter, all alkaline phosphatase treatment was carried out in the same manner). Ligation was then carried out, and transformation of *Escherichia coli* DH5α and inoculation on an LB+Amp plate were carried out, followed by culturing at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The prepared plasmid DNA was treated with a suitable restriction enzyme to inspect whether or not a DNA fragment had been inserted and the insertion direction, thereby selecting the plasmid having a fragment incorporated therein as expected. This plasmid was designated as "p5'-3'pBM/HCV."

### Construction of nE2

A transcription termination signal was inserted downstream of the HCV translated region in p5'-3' pBM/HCV, and CAG promoter was inserted upstream of the HCV translated region to realize the construction of pE2. The construction was performed in the following manner. At the outset, p5'-3'pBM/HCV and pCALN/HCV RBZ were respectively double-digested with *Xba*I and *Hind*III, followed by agarose electrophoresis. A 12.2 kbp DNA fragment of p5'-3'pBM/HCV and a 0.6 kbp DNA fragment of pCALN/HCV RBZ were extracted from a gel, and these DNA fragments were ligated. *Escherichia coli* DH5α was transformed with the ligated fragment, and inoculated on an LB+Amp plate, followed by culturing at 37°C overnight. The developed colonies were shake-cultured in a tube containing 3 mL of LB+Amp medium at 37°C overnight, the culture solution was centrifuged to harvest, and plasmid DNA was prepared by mini-preparation. This plasmid DNA was then partially cleaved with *Eco*RI in the following manner. 2.2 µg of the plasmid DNA was treated with 2.0 units of *Eco*RI at 37°C for 3 minutes, immediately immersed in ice water to stop the reaction, and subjected to agarose electrophoresis. A 12.7 kbp DNA fragment in which only one of two *Eco*RI sites on the vector had been cleaved was extracted from a gel. The extracted DNA fragment was then digested with *Xho*I, subjected to agarose electrophoresis, and a 12.7 kbp DNA fragment was extracted from a gel. As an insert DNA fragment, pCALN/HCV RBZ was digested with *Eco*RI and then partially cleaved with *Xho*I in the following manner. *Eco*RI-treated linear plasmid DNA (10 µg) was treated with 12 units of *Xho*I at 37°C for 3 minutes, immediately immersed in ice water to stop the reaction, and subjected to agarose electrophoresis. A 3.1 kbp DNA fragment in which only one of three *Xho*I sites on the vector had been cleaved was extracted from a gel. Subsequently, the 12.7 kbp DNA fragment and the 3.1 kbp DNA fragment were ligated. *Escherichia coli* DH5α was transformed with the ligated fragment, and inoculated on an LB+Amp plate, followed by culturing at 37°C overnight. The developed colonies were shake-cultured in a tube containing 3 mL of LB+Amp medium at 37°C overnight, the culture solution was centrifuged to harvest, and plasmid DNA was prepared by mini-preparation. The size of the plasmid was inspected by restriction enzyme treatment and, as a result, a plasmid having a construction as expected was obtained. This plasmid was designated as "pE2."

pE2 is a vector whose expression is controlled by the Cre-loxP system, as with pCALN/HCV RBZ. Thus, the vector can produce an expression system using switching expression.

### Construction of pE2b

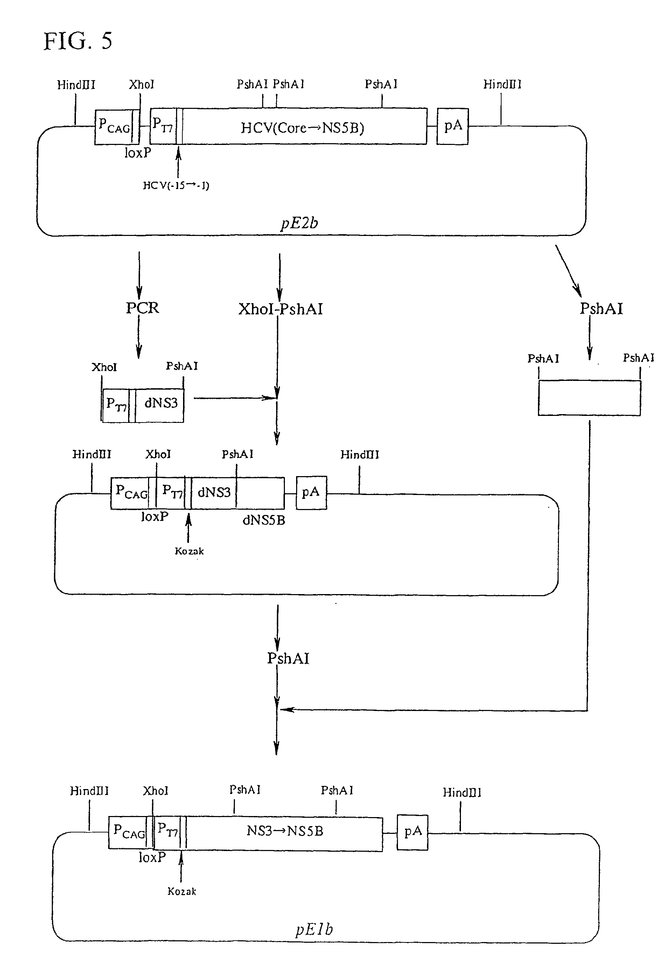

pE2b is a vector which was designed to bring about expression in the cell simultaneously with gene introduction on the assumption that the vector is used in an expression system employing no switching expression. pE2b was constructed based on pE2 in the following manner. The above pE2 was digested with *Xho*I and subjected to agarose electrophoresis, and a 14.5 kbp DNA fragment was extracted. The extracted fragment was subjected to self-ligation in a ligation solution. *Escherichia coli* DH5α was transformed with the resulting fragment, inoculated on an LB+Amp plate, and cultured at 37°C overnight. The developed colonies were shake-cultured in a tube containing 3 mL of LB+Amp medium at 37°C overnight, the culture solution was centrifuged to harvest, and plasmid DNA was prepared by mini-preparation. The size of the plasmid DNA was inspected by the restriction enzyme treatment and, as a result, an expected plasmid DNA was obtained. This plasmid DNA was then digested with *Hind*III and subjected to agarose electrophoresis, and an 11.4 kbp DNA fragment was extracted from a gel. pUC19 was digested with *Hind*III and subjected to agarose electrophoresis, and a 2.7 kbp linear plasmid DNA was extracted. The linear plasmid DNA was subjected to alkaline phosphatase treatment. This alkaline phosphatase-treated linear plasmid and the 11.4 kbp DNA fragment were ligated. *Escherichia coli* DH5α was transformed with the ligated fragment, inoculated on an LB+Amp plate, and cultured at 37°C overnight. The developed colonies were shake-cultured in a tube containing 3 mL of LB+Amp medium at 37°C overnight, the culture solution was centrifuged to harvest, and plasmid DNA was prepared by mini-preparation. The insertion direction of an insert was confirmed by a suitable restriction enzyme treatment, and a clone inserted in an opposite direction to Lac promoter of pUC19 was selected. This clone was designated as "pE2b" (Fig. 5).

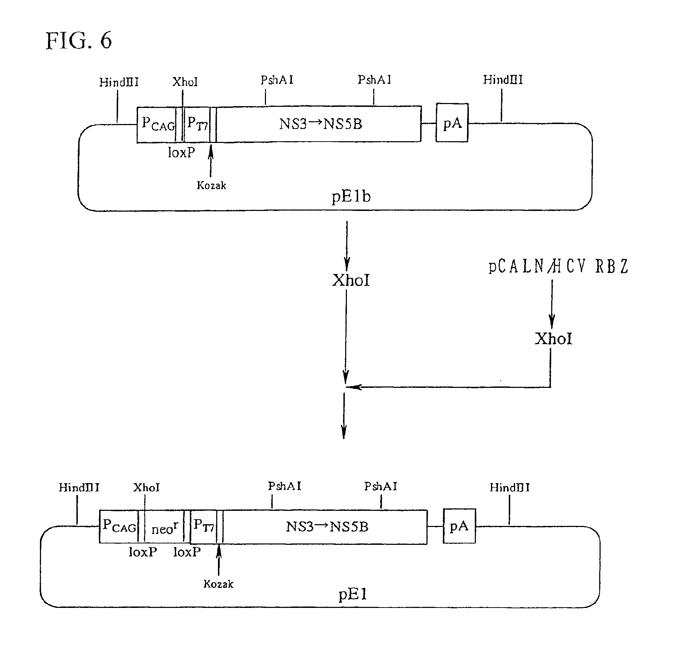

### 2) Construction of pE1b (Fig. 5) and pE1 (Fig. 6)

### Construction of pE1b

The HCV genomic RNA in the cell has been demonstrated to be replicated partially for only a non-structural protein, from NS3 to NS5B (Science 285; 110-113 (1999)). Thus, expression vectors pE1 and pE1b in the HCV translated region from NS3 to NS5B were constructed in parallel with the construction of the expression vectors pE2 and pE2b for the full length of the HCV translated region. As with pE2b, pE1b was constructed on the assumption that it was for use in an expression system employing no switching expression, and as with pE2, pE1 was constructed for producing an expression system employing switching expression. The procedure for constructing pE1b is shown below. PCR was first carried out using pE2 as a template. The sequences of the PCR primers are as follows.

NS3 Fw1: 5'-AAA CTC GAG TAA TAC GAC TCA CTA TAG GGC CAC CAT
GGC TCC CAT CAC GGC CTA TTC-3' (SEQ ID NO: 11)

NS3 Rev1: 5'-CCA TTG ACG CAG GTC GCC AG-3' (SEQ ID NO: 12)

Using Pyrobest DNA Polymerase (Takara Shuzo Co., Ltd.) to prepare a similar reaction solution as that described above, PCR was carried out through 15 cycles of denaturation at 98°C for 10 seconds, annealing at 62°C for 30 seconds, and elongation at 72°C for 30 seconds. The amplified sequence is shown in SEQ ID NO: 3. A part of this PCR product was subjected to 1.5% agarose electrophoresis. Upon confirmation of specific amplification of a fragment having a desired size, the PCR product was purified using PCR purification kit (QIAGEN) in the following manner. At the outset, PB Buffer of 5x the amount of the PCR reacted solution was added and thoroughly mixed. Thereafter, the mixture was transferred into QIAquick spin column, followed by centrifugation. The flow-through fraction was removed, and 750 µL of PE Buffer was added in the QIAquick spin column for washing, followed by centrifugation. The flow-through fraction was removed and centrifugation was carried out again to completely remove the PE Buffer. The contents of the QIAquick spin column was transferred into a 1.5 mL tube, sterilized water was added, and the mixture was centrifuged to elute DNA (hereinafter, all purification of DNA fragments is done in this manner). Subsequently, the DNA was double-digested with *Psh*AI and *Xho*l. The digest was then subjected to 2.0% agarose electrophoresis and a 0.2 kbp DNA fragment was extracted from a gel. pE2b was double-digested with *Psh*AI and *Xho*I in the restriction enzyme reaction solution, subjected to agarose electrophoresis, and 7.1 kbp and 3.8 kbp DNA fragments were extracted from a gel. A 7072 bp DNA fragment was ligated to the double-digested PCR product. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After incorporation of a DNA fragment was confirmed by restriction enzyme treatment, this plasmid DNA was digested with *Psh*AI and purified using the PCR purification kit, followed by alkaline phosphatase treatment. The treated DNA was purified again using the PCR purification kit and ligated to the 3780 bp DNA fragment. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The plasmid was designated as "pE1b."

### Construction of pE1

pE1 is a vector whose expression is controlled by the Cre-loxP system, as with pE2, and this vector enables the production of an expression system employing switching expression. pE1 was constructed based on pE1b in the following manner. At the outset, pE1b was digested with *Xho*I in a restriction enzyme solution. After DNA purification, the purified DNA was subjected to alkaline phosphatase treatment, and DNA was purified again. pCALN/HCV RBZ was digested with *Xho*I in the restriction enzyme solution and subjected to agarose electrophoresis, and a 1.2 kbp DNA fragment was extracted. These DNA fragments were ligated, and used to transform *Escherichia coli* DH5α. The *Escherichia coli* DH5α inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. Through the restriction enzyme treatment, a clone having a DNA fragment inserted in a positive direction was selected and designated as "pE1."

### (2) Construction of reporter gene expression vector

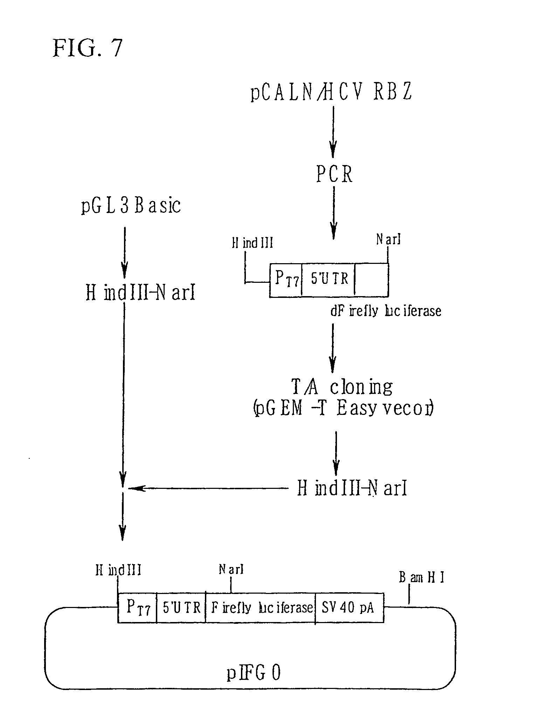

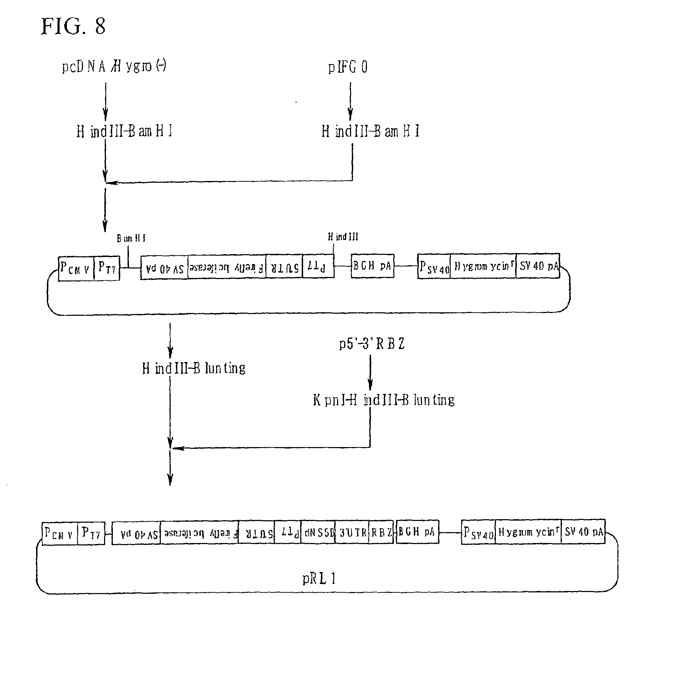

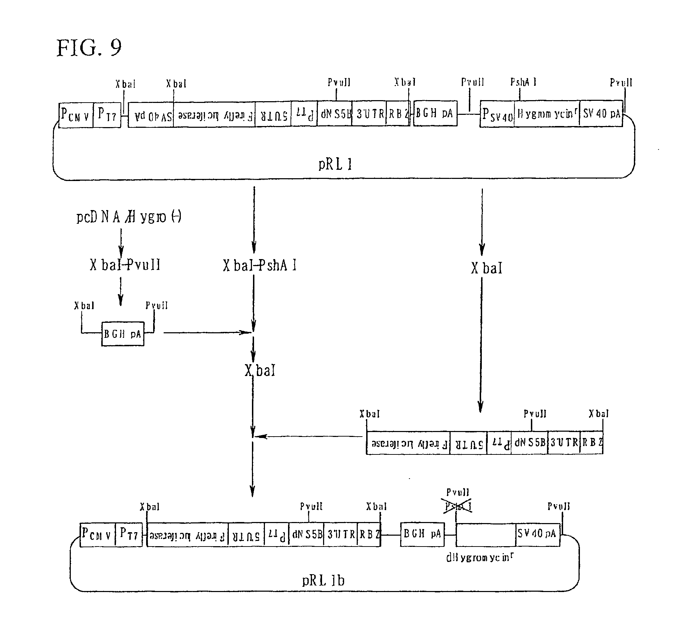

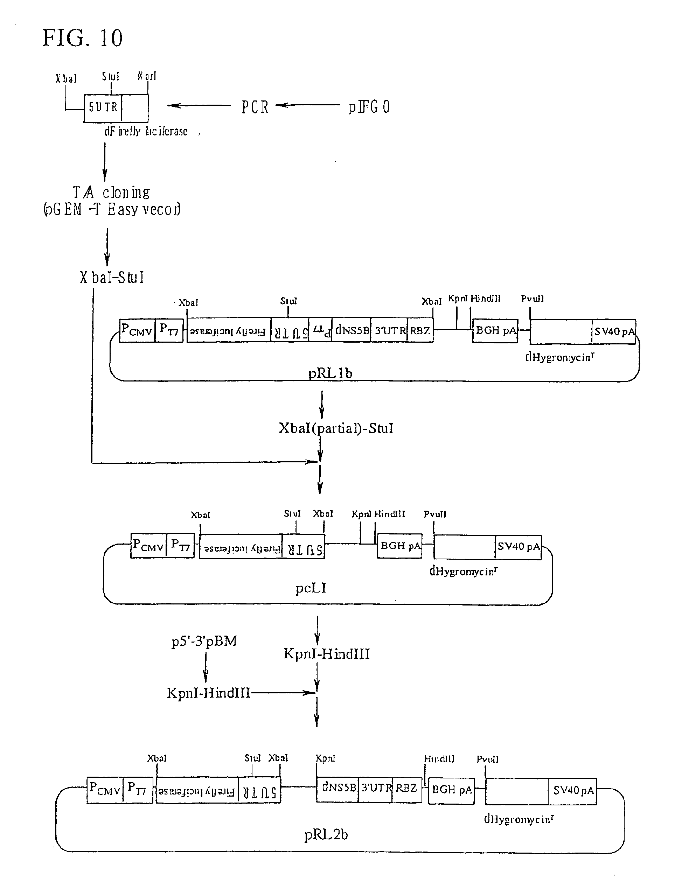

### 1) Construction of pRL2b (pIFGO: Fig. 7, pRL1: Fig. 8, pRL1b: Fig. 9, pRL2b: Fig. 10) Construction of pIFGO

At the outset, vector pIFGO comprising T7 promoter and HCV IRES linked upstream of the luciferase gene was constructed in the following manner. PCR was first carried out using pCALN/HCV RBZ as a template. The nucleotide sequences of PCR primers used are as follows.

IFGO: 5'-AAC TGC AGA AGC TTT AAT ACG ACT CAC TAT AGC CAG CC
C CCG ATT GGG G-3' (SEQ ID NO: 13)

IFRR: 5'-ATG GCG CCG GGC CTT TCT TTA TGT TTT TGG CGT CTT CCA
TGA TGC ACG GTC TAC GAG-3' (SEQ ID NO: 14)

The PCR reaction solution was prepared by adding 10 µL of 10x Buffer, 8 µL of 25 mM dNTP mixture, 2 µL each of 10 µM primer, 1 µL of 100 ng/µL pCALN/HCV RBZ, and 1.0 µL of 5 units/µL AmpliTaq DNA Polymerase (Perkin Elmer) to a tube and adjusting to 100 µL with the aid of sterilized water. In PCR, heating at 96°C for 5 minutes was first carried out, and a cycle of denaturation at 96°C for 30 seconds, annealing at 60°C for 15 seconds, and elongation at 72°C for 40 seconds was then repeated 25 times. The amplified sequence is shown in SEQ ID NO: 4. The PCR product was subjected to electrophoresis, and a specifically amplified fragment of interest was extracted from a gel. 1 µL thereof was used in T/A cloning, i.e., ligation to a T-vector. T/A cloning was carried out in the following manner. 1 µL of pGEM-T Easy Vector (Promega), 5 µL of 2x buffer, 1 µL of T4 DNA ligase, and 2 µL of sterilized water were added to 1 µL of the reaction solution in order to bring the total amount to 10 µL, and the mixture was incubated at room temperature for 1 hour. *Escherichia coli* DH5α was transformed with the product, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After checking by a suitable restriction enzyme treatment that insert DNA was inserted, the nucleotide sequence of the insertion part was analyzed to confirm it was the correct sequence. The confirmed plasmid was used below. This plasmid DNA was also double-digested with *Nar*I and *Hind*III. pGL3 Basic vector (Promega) was double-digested with *Nar*I and *Hind*III in the same manner. The reacted solution was subjected to agarose electrophoresis. For the PCR product, a 0.4 kbp DNA fragment was extracted from a gel, and for the pGL3 Basic vector, a 4.8 kbp DNA fragment was extracted from a gel. Subsequently, these two DNA fragments were ligated. *Escherichia coli* DH5α strain was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. Through the restriction enzyme treatment, insertion of a DNA fragment into a vector was inspected and confirmed. The resulting vector was designated as "pIFGO."

### Construction of pRL1

pRL1 was constructed as described below. The pIFGO was double-digested with *Bam*HI*-Hind*III and subjected to agarose electrophoresis, and a 2.3 kbp DNA fragment was extracted from a gel. pcDNA 3.1/Hygro (-) (Invitrogen) was double-digested with *Bam*HI*-Hind*III and subjected to agarose electrophoresis, and a DNA fragment was extracted from a gel. Subsequently, these DNA fragments were ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After insertion of a DNA fragment was confirmed by a restriction enzyme treatment, this plasmid was cleaved with *Hind*III. After purification, blunt-ending using Klenow Fragment (Takara Shuzo Co., Ltd.) was carried out in the following manner. 2.5 µL of 10x buffer, 2.5 µL of 2.0 mM dNTP, and 1.0 µL of Klenow Fragment were added to a suitable amount of DNA solution, and the solution was brought to 25 µL with the addition of sterilized water. The mixture was incubated at room temperature for 30 minutes, and a DNA fragment was then purified. As an insert, the p5'-3'RBZ was double-digested with *Kpn*I and *Hind*III and subjected to agarose electrophoresis, and a 0.6 kbp DNA fragment was extracted from a gel. Subsequently, the DNA fragment was blunt-ended using DNA blunting kit (Takara Shuzo Co., Ltd.) in the following manner. 9 µL of DNA solution and 1 µL of 10x buffer (attached to the kit) were mixed, and the mixture was incubated at 70°C for 5 minutes and at 37°C for 1 minute. Thereafter, 1 µL of T4 DNA polymerase was added, the resultant mixture was incubated at 37°C for 5 minutes, and a DNA fragment was then purified. Subsequently, this DNA fragment was subjected to alkaline phosphatase treatment, and a DNA fragment was again purified. These DNA fragments were then ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After insertion of a DNA fragment was confirmed by a suitable restriction enzyme treatment, a nucleotide sequence of the ligated portion was analyzed. As a result, it was confirmed to be the expected sequence. The constructed plasmid was designated as "pRL1."

### Construction of pRL1b

In the above pRL1, a full length SV40 origin exists downstream of the reporter expression unit. Thus, the possibility that transcription may occur upstream of the SV40 late promoter in that portion was foreseen, and there was a concern that it would become a background in the assay of this reporter gene replication system. Accordingly, improved vector pRL1b was constructed in which the SV40 origin was removed. The improved vector pRL1b was constructed in the following manner. pRL1 was first double-digested with *Psh*AI and *Xba*I, and a 4.4 kbp DNA fragment was extracted. pcDNA3.1/-Hygro(-) was double-digested with *Pvu*II and *Xba*I and a 0.4 kbp DNA fragment was extracted. These extracted DNA fragments were ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After insertion of a DNA fragment was confirmed by a suitable restriction enzyme treatment, this plasmid was digested with *Xba*I and purified, followed by alkaline phosphatase treatment. The treated plasmid was purified again. As insert DNA, pRL1 was digested with *Xba*I, subjected to agarose electrophoresis, and a 2.6 kbp DNA fragment was extracted from a gel. This DNA fragment was ligated to the *Xba*I-digested plasmid DNA. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The insertion direction of the DNA fragment was inspected by a suitable restriction enzyme treatment. A plasmid having the insert in a targeted direction was selected and designated as "pRL1b." This pRL1b is a plasmid in which the SV40 origin has been removed and SV40 late poly (A) signal downstream of the reversed luciferase gene has also been removed.

### Construction of pRL2b

In order to perform examination in an overexpression system using vaccinia viruses (T7VV) coding for T7 RNA polymerase, vector pRL2b was constructed in which T7 promoter immediately before the reversed 5'UTR sequence on pRL1b was removed.

In pRL1b, T7 promoters are disposed on both sides of <HCV 5'UTR-luciferase> which is inversely inserted downstream of CMV promoter. Thus, when attempting to excessively synthesize the anti-sense strand RNA (hereinafter referred to as "(+) strand RNA") of the luciferase gene with T7 RNA polymerase, sense strand RNA (hereinafter referred to as "(-) strand RNA") of the luciferase gene is simultaneously synthesized, and, based on the RNA, luciferase is disadvantageously translated. Thus, it is unsuitable for evaluating the (-) strand RNA synthesis by NS5B. pRL2b is a vector that was reconstructed in order to keep the T7 promoter immediately downstream of the CMV promoter and to remove the T7 promoter downstream of the reversed <HCV 5'UTR-luciferase>. pRL2b was constructed as described below. At the outset, PCR was carried out using pIFGO as a template to remove T7. The nucleotide sequence of the Forward primer used in PCR is shown below. The IFRR was used as the Reverse primer.

DLIR1:5'-GCT CTA GAC AGG GCC AGC CCC CGA TTG-3' (SEQ ID NO: 15)

The PCR reaction solution was prepared by adding 10 µL of 10x Buffer, 8 µL of 25 mM dNTP mixture, 2 µL each of 10 µM primer, 1 µL of 100 ng/µL pIFGO, and 1.0 µL of 5 units/µL AmpliTaq DNA Polymerase (Perkin Elmer) to a tube and adjusting to 100 µL with the aid of sterilized water. In PCR, heating at 96°C for 5 minutes was first carried out, and a cycle of denaturation at 96°C for 30 seconds, annealing at 60°C for 15 seconds, and elongation at 72°C for 40 seconds was then repeated 25 times. The amplified sequence is shown in SEQ ID NO: 5. The PCR product was subjected to electrophoresis, and a specifically amplified fragment of interest was extracted from a gel. 1 µL thereof was used in T/A cloning, i.e., ligation to the T-vector. T/A cloning was carried out in the above-described manner. *Escherichia coli* DH5α was transformed with the ligated solution, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After checking by a suitable restriction enzyme treatment that insert DNA was inserted, the nucleotide sequence of the insertion part was analyzed to confirm it was the correct sequence. The confirmed plasmid was used below. This plasmid was double-digested with *Xba*I*-Stu*I, and a 0.3 kbp DNA fragment was extracted from a gel. pRL1b was partially cleaved with *Xba*I as described below. 2 µg of pRL1b was treated with 3 units of *Xba*I at 37°C for 20 seconds and immediately immersed in ice water to terminate the reaction. The reaction product was then subjected to agarose electrophoresis, and a 7.4 kbp DNA fragment in which only one of two *Xba*I sites on the vector had been cleaved was extracted from a gel. The extracted DNA fragment was then digested with *Stu*I. A 6.5 kbp DNA fragment was then extracted from a gel. These DNA fragments were ligated, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The insertion direction of the DNA fragment was inspected by a suitable restriction enzyme treatment. A plasmid having the insert in a targeted direction was selected, designated as "pcLI," and put to use in the operation below. pcLI was double-digested with *Kpn*I and *Hind*III and subjected to agarose electrophoresis, and a 6.7 kbp DNA fragment was extracted from a gel. As an insert, p5'-3'RBZ was double-digested with *Kpn*I and *Hind*III and subjected to agarose electrophoresis, and a 0.6 kbp DNA fragment was extracted from a gel. These DNA fragments were ligated. *Escherichia coli* DH5α was transformed with the ligated fragment, inoculated on an LB+Amp plate, and cultured at 37°C overnight. The colonies developed on the plate were inoculated on an LB+Amp medium and then cultured at 37°C overnight, and plasmid was prepared by mini-preparation. Through double digestion with *Kpn*I and *Hind*III, a plasmid having inserted DNA incorporated therein as expected was selected and designated as "pRL2b."

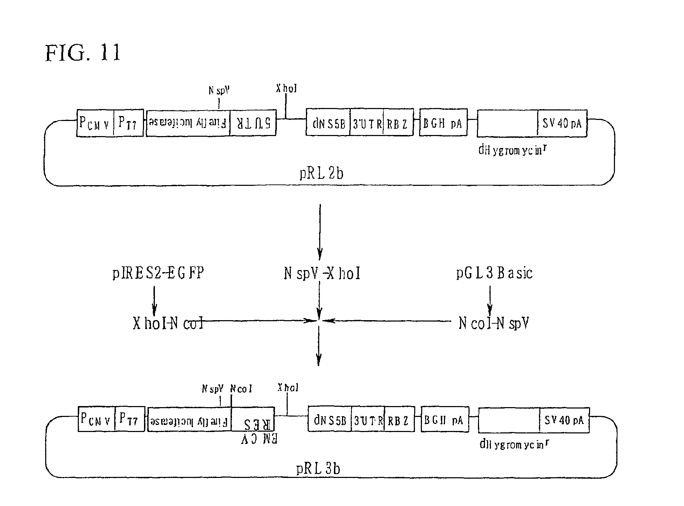

### Construction of pRL3b (Fig. 11)

In pRL2b, HCV IRES is used as IRES for translating luciferase proteins. In order to enhance translation efficiency and detection sensitivity for luciferase activity, vector pRL3b in which the HCV IRES was replaced with EMCV IRES was constructed as described below. At the outset, pRL2b was double-digested with *Xho*I and *Nsp*V and subjected to electrophoresis, and a 6.8 kbp DNA fragment was extracted from a gel. Subsequently, pGL3 Basic vector (Promega) was double-digested with *Nco*I and *Nsp*V and subjected to electrophoresis, and a 0.2 kbp DNA fragment was extracted from a gel. pEIRES2-EGFP (CLONTECH) was then double-digested with *Xho*I and *Nco*I and subjected to electrophoresis, and a 0.6 bp DNA fragment was extracted from a gel. These 3 extracted DNA fragments were ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. Through triple digestion with *Xho*I, *Nco*I, and *Xba*I, whether or not insert DNA had been incorporated was inspected, and a vector having insert DNA incorporated as expected was designated as "pRL3b."

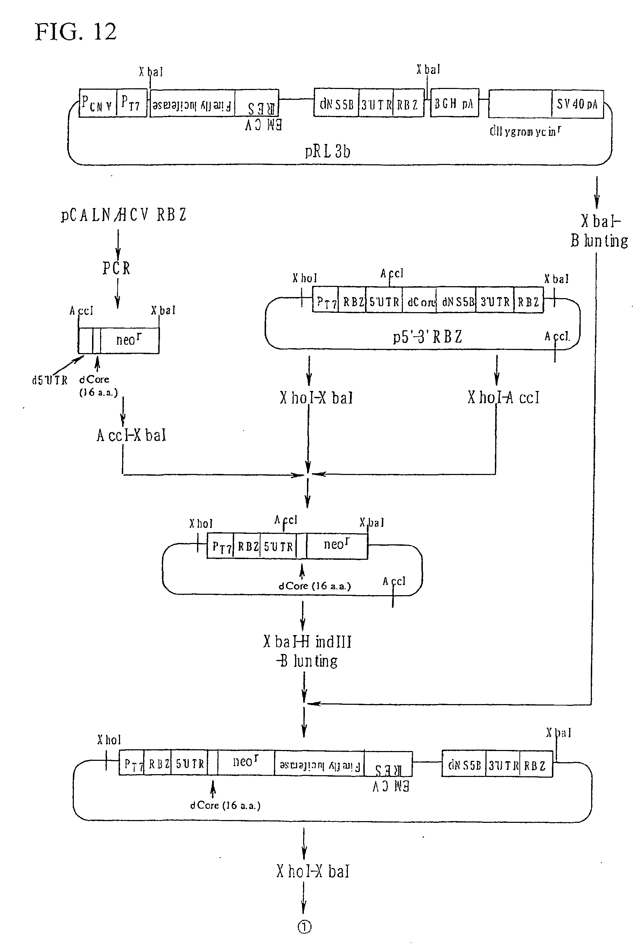

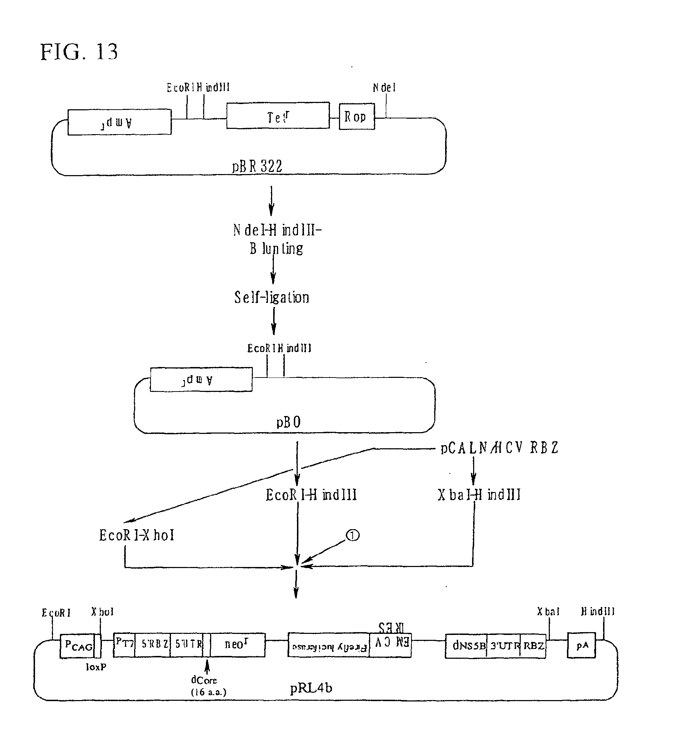

### Construction of pRL4b (Figs. 12 and 13)

With the addition of HCV 5'UTR to the 5' terminus of the (+) strand RNA molecule synthesized from pRL3b, the structure of the vector becomes closer to that of the original HCV genome. As a result, replication efficiency was expected to improve and reporter activity to be enhanced. The reason being that since 5'UTR as well as 3'UTR are cooperatively associated with the function of HCV IRES (J. Virol. 72; 8789-8796 (1998)), the possibility that 5'UTR cooperatively acts with 3'UTR in RNA synthesis was also considered. Thus, a reporter expression vector pRL4b was constructed in which HCV 5'UTR and the nucleotide sequence corresponding to the N-terminal 16 amino acids of a core protein have been added to the 5' terminus of the (+) strand RNA molecule. Referring to a report in the journal "Science" (Science 285; 110-113 (1999)), a neomycin-resistant gene was connected to the nucleotide sequence corresponding to the N-terminal 16 amino acids. This was done on the assumption that various examinations as described below would be carried out later. For example, it was considered that when RNA transfection similar to that reported in the above "Science" journal is carried out, more specifically, when RNA synthesized *in vitro* using pRL4b as a template and T7 RNA polymerase is transfected into a strain having stably introduced pE1 or pE2, continuation of culture in the presence of neomycin under the induction of HCV-derived protein expression may result in replication of transfected RNA molecules by HCV-derived protein and the possible eventual selection of a neomycin-resistant clone.

The process for constructing pRL4b is shown below. In order to obtain a ligated DNA fragment containing 5'UTR and portions from the nucleotide sequence corresponding to the N-terminal 16 amino acids to the neomycin-resistant gene terminus, PCR was first carried out using pCALN/HCV RBZ as a template. The nucleotide sequences of the PCR primers used are as follows.

neo Fw:5'-GTA GAC CGT GCA TCA TGA GCA CAA ATC CTA AAC CCC
AAA GAA AAA CCA AAC GTA ACA CCA ACA TTG AAC AAG ATG GAT
TGC ACG C-3' (SEQ ID NO: 16)

neo Rv:5'-TCT AGA TTA TCA GAA GAA CTC GTC AAG AAG GCG -3'
(SEQ ID NO: 17)

The PCR reaction solution was prepared in the same manner as described above using Pyrobest DNA Polymerase (Takara Shuzo Co., Ltd.). In PCR, heating at 94°C for 5 minutes was first carried out, and a cycle of denaturation at 98°C for 10 seconds, annealing at 60°C for 30 seconds, and elongation at 72°C for 1 minute and 20 seconds was then repeated 20 times. The amplified sequence is shown in SEQ ID NO: 6. The PCR product was then subjected to 1.0% agarose electrophoresis, and a DNA fragment of a desired size was extracted from a gel. Subsequently, "A" was added to the 3' terminus of the fragment in the following manner in order to carry out T/A cloning. 1 µL of recombinant Taq DNA polymerase (Takara Shuzo Co., Ltd.), 1 µL of 10x buffer, 0.8 µL of 2.5 mM dATP, and 1 µL of 25 mM MgCl₂ were added to a suitable amount of DNA solution, and the total amount of the solution was brought to 10 µL with the addition of sterilized water. This solution was incubated at 70°C for 30 minutes. 1 µL of the reaction solution was used in T/A cloning. T/A cloning was carried out in the above-described manner. *Escherichia coli* DH5α was transformed with the ligated solution, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. After checking by a suitable restriction enzyme treatment that insert DNA was inserted, the nucleotide sequence of the insertion part was analyzed to confirm it was the correct sequence. The confirmed plasmid was used below. This plasmid was double-digested with *Acc*I and *Xba*I, and the resultant 0.9 kbp DNA fragment was extracted from a gel. p5'-3'RBZ was double-digested with *Xho*I and *Acc*I, and separately double-digested with *Xho*I and *Xba*I. Regarding the former, a 0.4 kbp DNA fragment was extracted from a gel and, regarding the latter, a 3.1 kbp DNA fragment was extracted from a gel. These 3 extracted DNA fragments were ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. Plasmid DNA having insert DNA incorporated therein was selected through a suitable restriction enzyme treatment. Subsequently, this plasmid DNA was cleaved with *Xba*I and purified. Thereafter, the plasmid DNA was blunt-ended using Klenow Fragment (in the same manner as described above), followed by alkaline phosphatase treatment. Separately, as an insert, the pRL3b was cleaved with *Xba*I, and subjected to agarose electrophoresis. A 3.0 kbp DNA fragment was then extracted and blunt-ended using Klenow Fragment (in the same manner as described above). These DNA fragments were ligated. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The insertion direction of the inserted DNA fragments was inspected through a suitable restriction enzyme treatment, and a clone having the insert in a targeted direction was used below. The plasmid obtained from this clone was double-digested with *Xho*I and *Xba*I and subjected to agarose electrophoresis, and a 4.1 kbp DNA fragment was extracted from a gel. Separately, pBR322 was double-digested with *Nde*I and *Hind*III and subjected to electrophoresis. A 2.1 kbp DNA fragment was then extracted from a gel and blunt-ended using Klenow Fragment (in the same manner as described above), and subjected to self-ligation in a ligation solution. *Escherichia coli* DH5α was transformed with the ligated solution, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. This vector was double-digested with *Eco*RI and *Hind*III and subjected to agarose electrophoresis, and a 2.1 kbp DNA fragment extracted from a gel was used as a vector. As inserts, a 0.6 kbp DNA fragment which was prepared by double-digesting pCALN/HCV RBZ with *Xba*I and *Hind*III, subjecting to agarose electrophoresis, and extracting from a gel, and a 1.8 kbp DNA fragment, which was prepared by double-digesting pCALN/HCV RBZ with *Eco*RI and *Xho*I, subjecting to agarose electrophoresis, and extracting from a gel, were used. The 4.1 kbp, 0.6 kbp, and 1.8 kbp inserts were ligated to the 2.1 kbp vector. *Escherichia coli* DH5α was transformed with the ligated vector, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The clone having three inserts inserted as targeted was confirmed by a suitable restriction enzyme treatment, and this clone was designated as "pRL4b."

### (3) Construction of cis-acting expression vector

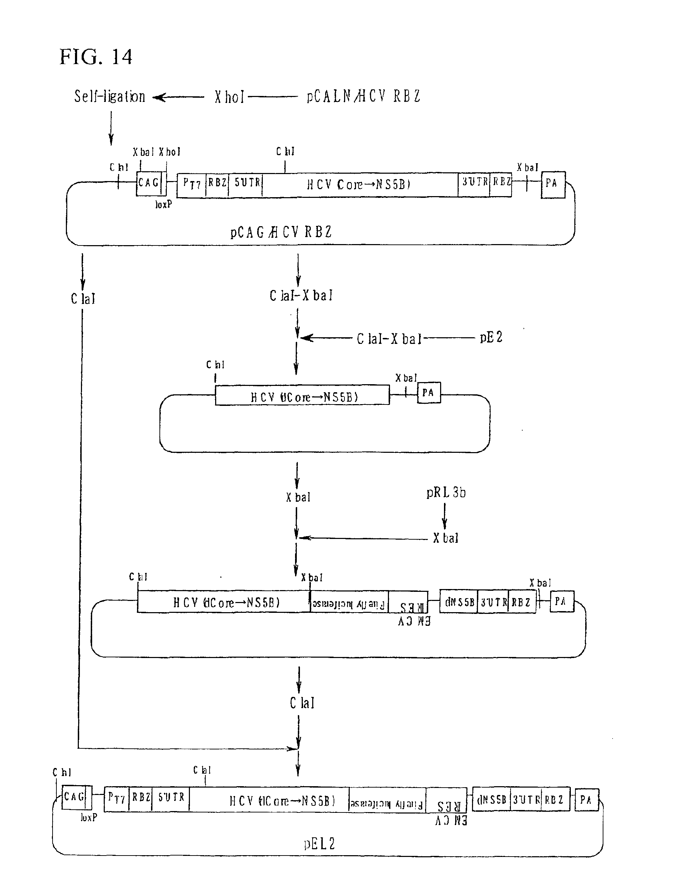

### 1) Construction of pEL2 (Fig. 14)

A cis-acting expression vector pEL2 was constructed as described below. pCALN/HCV RBZ was first digested with *Xho*I and subjected to electrophoresis. A 16.5 kbp DNA fragment was extracted and subjected to self-ligation in a ligation solution. *Escherichia coli* DH5α was transformed with the ligated fragment, inoculated on an LB+Amp plate, and cultured at 37°C overnight. The colonies developed on the plate were shake-cultured in a tube containing 3 mL of LB+Amp medium at 37°C overnight, the culture solution was centrifuged to harvest, and plasmid DNA was prepared by mini-preparation. The size of the plasmid DNA was inspected and the one with the correct size was designated as "pCAG/HCV RBZ." Subsequently, this pCAG/HCV RBZ was double-digested with *Cla*I and *Xba*I and subjected to electrophoresis, and a 4.9 kbp DNA fragment was extracted and determined as vector DNA. pE2 was double-digested with *Cla*I and *Xba*I and subjected to electrophoresis, and a 8.7 kbp DNA fragment was extracted for use as an insert DNA. These DNA fragments were ligated, and *Escherichia coli* was transformed with the ligated fragment. The colonies developed on the plate were shake-cultured in a tube containing 3 mL of LB+Amp medium at 37°C overnight, the culture solution was centrifuged to harvest, and plasmid DNA was prepared by mini-preparation. Subsequently, the resultant plasmid DNA was digested with *Xba*I and purified with PCR purification kit, followed by BAP treatment. On the other hand, the pRL3b was treated with *Xba*I and subjected to electrophoresis, and a 3.0 kbp DNA fragment was extracted for use as an insert. These DNA fragments were ligated, and *Escherichia coli* DH5α was transformed with the ligated fragment, and cultured at 37°C overnight. Several colonies among the obtained colonies were inoculated on LB+Amp medium and shake-cultured at 37°C overnight, and plasmids were prepared by mini-preparation. The insertion direction of the inserts was inspected by a suitable restriction enzyme treatment, and a plasmid for which incorporation was confirmed to be in the expected direction was digested with *Cla*I and purified by PCR purification kit, followed by BAP treatment. Separately, the pCAG/HCV RBZ was digested with *Cla*I and a 2.6 kbp DNA fragment was extracted as an insert. These fragments were ligated, and *Escherichia coli* was transformed with the ligated fragment. The colonies developed on the plate were shake-cultured in a tube containing 3 mL of LB+Amp medium at 37°C overnight, the culture solution was centrifuged to harvest, and plasmid DNA was prepared by mini-preparation. The insertion direction of the inserts was inspected by a suitable restriction enzyme treatment, and a plasmid for which incorporation was confirmed to be in the expected direction was designated as "pEL2."

### [Example 2]

### (1) Transfection into cell

Transfection of each vector into a cell was performed by the calcium phosphate method. Each DNA solution was mixed with a 0.2M calcium chloride solution, an equivalent volume of 2x HBS solution was added thereto, and the mixture was allowed to stand at room temperature for 10 minutes. Thereafter, a culture solution was added thereto, and the mixture was added to the IMY cell, which was inoculated at a concentration of 1.5 to 2.0 x 10⁵ cells/ml on the previous day. The input amount of each vector was 4 µg/well. After culturing at 37°C under 5% CO₂ for 4 hours, the mixture was washed with a culture solution several times, and when transcription was not induced by T7 RNA polymerase-expressing vaccinia viruses (T7VV), a new culture solution was added and then cultured for 2 days. When transcription was induced by T7VV, viruses were added at MOI of 10, and after adsorption for 1 hour, a new culture solution was added, followed by culturing for 10 hours. After culturing and washing with PBS (-), cells were peeled using a cell scraper, put into a 1.5 mL tube, and stored at -80°C until subjection to luciferase assay.

### (2) Luciferase assay

Luciferase assay was carried out using Luciferase Assay systems (Promega) as described below. 5x Cell Culture Lysis Reagent was diluted 5-fold with sterilized water, 250 µL thereof was fractionated into a 1.5 mL tube containing the above-described cells, and the cells were suspended. Centrifugation was carried out to remove cell debris, and the supernatant was subjected to luciferase assay as a lysate. In the assay, 20 µL of lysate was fractionated into a 96-well plate (OPAQUE PLATE (COSTAR)), 100 µL of substrate solution was added, and each well was measured for 10 seconds using MICROLUMAT LB96P (Berthold). The results of measurement are shown in Table 1 and Table 2.

**Table 1**

| Vector | Luciferase activity | Relative value ** |
|---|---|---|
| pRL2b | 521 | 1.0 |
| pRL2b+pE1b | 3,460 | 6.6 |
| pRL2b+pE2b | 7,317 | 14.0 |
| pRL3b | 6,183 | 1.0 |
| pRL3b+pE1b | 48,430 | 7.8 |
| pRL3b+pE2b | 35,865 | 5.8 |
| Control 1* | 104 | - |
| Control 2* | 90 | - |

| | | |
|---|---|---|
| *) Control 1: A vector is not introduced and transcription is induced by T7VV. | | |
| Control 2: A vector is not introduced and transcription is not induced by T7VV. | | |
| **) Relative value based on the luciferase activity (1.0) in the case where only a reporter gene expression vector is introduced. | | |

**Table 2**

| Vector | No transcription induced by T7VV | | Transcription induced by T7VV | |
|---|---|---|---|---|
| | Luciferase activity | Relative value** | Luciferase activity | Relative value** |
| pRL3b | 1,609 | 1.0 | 28,530 | 1.0 |
| pRL3b+pE1b | 4,127 | 2.6 | 487,409 | 17.1 |
| pRL3b+pE2b | 10,607 | 6.6 | 182,718 | 6.4 |
| pRL4b | 315 | 1.0 | 8,456,230 | 1.0 |
| pRL4b+pE1b | 747 | 2.4 | 20,673,314 | 2.4 |
| pRL4b+pE2b | 995 | 3.2 | 14,430,188 | 1.7 |
| pEL2 | 331 | - | 11,704,062 | - |
| Control 1* | 80 | - | 112 | - |

| | | | | |
|---|---|---|---|---|
| *) No vector introduced. | | | | |
| **) Relative value based on the luciferase activity (1.0) in the case where only a reporter gene expression vector is introduced. | | | | |

Table 1 shows the results of double transfection of pRL2b or pRL3b and pE1b or pE2b, which are trans-acting vectors. In the table, the results of transfection of the reporter gene expression vector alone is set as a background level, and change in the luciferase activity in the presence of an enzyme expression vector is inspected. Table 2 shows the results of double transfection of pRL3b or pRL4b and pE1b or pE2bb, which are trans-acting vectors. Further, a cis-acting vector pEL2 was simultaneously inspected.

Table 1 and Table 2 demonstrate the following.
a) For pRL2b, pRL3b, and pRL4b, co-expression of enzyme expression vectors results in increase in the reporter activity. Thus, (-) strand RNA is considered to be synthesized by NS5B. Although activity was also observed when only the reporter gene expression vector was introduced into the cell, this is considered to be a leak derived from (-) strand RNA synthesized by a promoter-like sequence downstream of the luciferase gene.
b) On the assumption that the amounts of (-) strand RNA synthesized by pRL2b and by pRL3b are the same, differences in the luciferase activity are considered to reflect the differences in the translation activity between HCV IRES and EMCV IRES.
c) Increase in the luciferase activity was observed in the cis-acting vector pEL2, suggesting that the synthesis of (-) strand RNA by NS5B occurs.

### Industrial Applicability

The present invention provides a novel vector for analyzing the replication mechanism of RNA viruses. This vector enables the evaluation of activity of RNA virus-derived RNA-dependent RNA polymerase based on the expression level of a reporter gene by taking the interaction between the viral protein based on the original life cycle of viruses and host factors into consideration.

All publications, patents and patent applications cited herein are incorporated herein by reference in their entirety.

## Claims

1. A vector for analyzing the replication mechanism of RNA viruses, which comprises DNA coding for RNA virus-derived RNA-dependent RNA polymerase.

2. A vector for analyzing the replication mechanism of RNA viruses, which comprises a reporter gene expressed in the anti-sense direction.

3. A vector for analyzing the replication mechanism of RNA viruses, which comprises DNA coding for RNA virus-derived RNA-dependent RNA polymerase and a reporter gene expressed in the anti-sense direction.

4. A kit for analyzing the replication mechanism of RNA viruses, which comprises the vector for analyzing the replication mechanism of RNA viruses according to claim 1 and the vector for analyzing the replication mechanism of RNA viruses according to claim 2.

5. A method for evaluating RNA polymerase activity, wherein a vector comprising DNA coding for RNA virus-derived RNA-dependent RNA polymerase and a vector comprising DNA corresponding to an entry site of the RNA polymerase and a reporter gene located upstream of the DNA and expressed in the anti-sense direction are produced, these vectors are introduced into a host cell, and the amount of the expression product of the reporter gene in the cell is measured, thereby evaluating RNA polymerase activity.

6. A method for evaluating RNA polymerase activity, wherein a vector comprising DNA coding for RNA virus-derived RNA-dependent RNA polymerase, DNA corresponding to an entry site of the RNA polymerase, and a reporter gene located upstream of the DNA corresponding to the entry site and expressed in the anti-sense direction is produced, this vector is introduced into a host cell, and the amount of the expression product of the reporter gene in the cell is measured, thereby evaluating RNA polymerase activity.

7. A method for screening a therapeutic agent for viral diseases, wherein a vector comprising DNA coding for RNA virus-derived RNA-dependent RNA polymerase and a vector comprising DNA corresponding to an entry site of the RNA polymerase and a reporter gene located upstream of the DNA and expressed in the anti-sense direction are produced, these vectors are introduced into a host cell, test substances are inoculated into this cell, and the amount of the expression product of the reporter gene in the cell is measured, and a substance which can increase or decrease the amount of the expression product of the reporter gene is selected from the test substances.

8. A method for screening a therapeutic agent for viral diseases, wherein a vector comprising DNA coding for RNA virus-derived RNA-dependent RNA polymerase, DNA corresponding to an entry site of the RNA polymerase, and a reporter gene located upstream of the DNA corresponding to the entry site and expressed in the anti-sense direction is produced, this vector is introduced into a host cell, test substances are inoculated into this cell, and the amount of the expression product of the reporter gene in the cell is measured, and a substance which can increase or decrease the amount of the expression product of the reporter gene is selected from the test substances.

9. A substance, which is obtained by the method for screening a therapeutic agent for viral diseases according to claim 7 or 8.

10. A cell, which comprises the vector according to claim 1 and the vector according to claim 2.

11. A cell, which comprises the vector according to claim 3.

12. A method for evaluating the replication capacity of RNA viruses, which comprises the steps of:
(1) expressing RNA virus-derived RNA-dependent RNA polymerase in a host;
(2) bringing RNA having a reporter gene located so as not to be expressed in the absence of RNA polymerase into contact with the RNA virus-derived RNA-dependent RNA polymerase; and
(3) measuring the expression level of the expression product of the reporter gene.

13. A method for evaluating the replication capacity of RNA viruses, which comprises the steps of:
(1) expressing RNA virus-derived RNA-dependent RNA polymerase in a host;
(2) expressing RNA coding for a reporter gene in the anti-sense direction;
(3) synthesizing RNA coding for the reporter gene in the sense direction by the RNA polymerase; and
(4) measuring the amount of the expression product of the reporter gene.

14. A method for evaluating the replication capacity of RNA viruses, which comprises the steps of:
(1) expressing a reporter gene incorporated in a first vector in the anti-sense direction and RNA coding for RNA-dependent RNA polymerase gene incorporated in a second vector that is the same as or different from the first vector in the sense direction in a host;
(2) expressing RNA-dependent RNA polymerase from the RNA;
(3) synthesizing RNA coding for the reporter gene in the sense direction by the RNA-dependent RNA polymerase; and
(4) measuring the amount of the expression product of the reporter gene.

15. A method for screening an inhibitor against viral replication comprising addition of a step of bringing a test substance into contact to the method according to any one of claims 12 to 14.

16. The vector according to any one of claims 1 to 3, wherein the RNA virus is HCV.

17. The method according to any one of claims 5, 6, and 12 to 14, wherein the RNA virus is HCV.

18. The method according to any one of claims 7, 8, and 15, wherein the RNA virus is HCV.

19. The cell according to claim 10 or 11, wherein the RNA virus is HCV.
